(19) Europäisches Patentamt / European Patent Office / Office européen des brevets

(11) **EP 3 295 178 B1**

(12) **FASCICULE DE BREVET EUROPEEN**

(45) Date de publication et mention de la délivrance du brevet:
**10.07.2019 Bulletin 2019/28**

(21) Numéro de dépôt: **16729013.9**

(22) Date de dépôt: **11.05.2016**

(51) Int Cl.:
*G01N 33/68* (2006.01)

(86) Numéro de dépôt international:
**PCT/FR2016/051098**

(87) Numéro de publication internationale:
**WO 2016/181066 (17.11.2016 Gazette 2016/46)**

(54) **PREDICTION DU RISQUE DE DEVELOPPER, POUR DES PATIENTS ADMIS EN SERVICE DE REANIMATION, UNE INFECTION DISSEMINEE**

VORHERSAGE DES RISIKOS EINER DISSEMINIERTEN INFEKTION VON AUF DER INTENSIVSTATION AUFGENOMMENEN PATIENTEN

PREDICTION FOR PATIENTS ADMITTED TO INTENSIVE CARE OF THE RISK OF DEVELOPING DISSEMINATED INFECTION

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **12.05.2015 FR 1554227**

(43) Date de publication de la demande:
**21.03.2018 Bulletin 2018/12**

(73) Titulaires:
- **bioMérieux**
  **69280 Marcy-l'Étoile (FR)**
- **Hospices Civils De Lyon**
  **69002 Lyon (FR)**
- **Centre Hospitalier Universitaire De Nantes**
  **44093 Nantes Cedex 1 (FR)**

(72) Inventeurs:
- **ALLAOUCHICHE, Bernard**
  **69008 Lyon (FR)**
- **ASEHNOUNE, Karim**
  **44000 Nantes (FR)**
- **DUPIN, Marilyne**
  **69670 Vaugneray (FR)**
- **FORTIN, Tanguy**
  **69006 Lyon (FR)**
- **GOUEL-CHERON, Aurélie**
  **75010 Paris (FR)**
- **LARUE-TRIOLET, Audrey**
  **69160 Tassin la Demi Lune (FR)**
- **MONNERET, Guillaume**
  **69003 Lyon (FR)**
- **PACHOT, Alexandre**
  **01400 Sulignat (FR)**
- **PONS, Sylvie**
  **69290 Saint Genis Les Ollieres (FR)**
- **ROQUILLY, Antoine**
  **Caulfield North, Victoria 3161 (AU)**
- **VENET, Fabienne**
  **69008 Lyon (FR)**

(56) Documents cités:
**WO-A2-2013/138800**

- **XIANHUI L. ET AL.: "The association between plasma gelsolin level and prognosis of burn patients", BURNS, vol. 40, no. 8, 30 mars 2014 (2014-03-30), pages 1552-1555, XP055232981,**
- **HUANG L.-F. ET AL.: "Reduction of plasma gelsolin levels correlates with development of multiple organ dysfunction syndrome and fatal outcome in burn patients", PLOS ONE, vol. 6, no. 11, E25748, 1 novembre 2011 (2011-11-01), pages 1-9, XP55291052,**
- **MOUNZER K.C. ET AL.: "Relationship of admission plasma gelsolin levels to clinical outcomes in patients after major trauma", AM. J. RESPIR. CRIT. CARE MED., vol. 160, no. 5(Pt1), novembre 1999 (1999-11), pages 1673-1681, XP002582956,**
- **BUCKI R. ET AL.: "Plasma gelsolin: function, prognostic value, and potential therapeutic", CURR. PROT. PEPT. SCI., vol. 9, no. 6, décembre 2008 (2008-12), pages 541-551, XP008122005,**

- WANG H.-H. ET AL.: "Time course of plasma gelsolin concentrations during severe sepsis in critically ill surgical patients", CRIT. CARE, vol. 12, no. 4, R106, 17 août 2008 (2008-08-17), pages 1-6, XP021046892, cité dans la demande

- LEE P.-S. ET AL.: "Plasma gelsolin depletion and circulating actin in sepsis - A pilot study", PLOS ONE, vol. 3, no. 11, E3712, 12 novembre 2008 (2008-11-12), pages 1-5, XP055232990, cité dans la demande

## Description

La présente invention concerne le domaine des maladies infectieuses. Plus particulièrement, la présente invention concerne une méthode de prédiction du risque de développer, pour des patients admis en service de réanimation, une infection disséminée, ou syndrome septique, par l'utilisation de la gelsoline en tant que biomarqueur.

Le syndrome septique, réponse systémique à une infection, également appelé infection disséminée, représente l'une des premières causes de mortalité dans les services de réanimation. En effet, les patients pris en charge dans les services de réanimation, sont des patients vulnérables présentant ou susceptibles de présenter une ou plusieurs défaillances viscérales aiguës engageant directement leur pronostic vital. Les causes pouvant conduire un patient en réanimation sont nombreuses : états de choc, polytraumatismes, comas, insuffisances organiques aiguës, décompensations de maladies chroniques, hémorragies, intoxications, infections sévères, brûlures étendues, période post-opératoire de chirurgies lourdes, etc. De façon générale, les patients qui requièrent des soins de réanimation sont atteints de défaillances organiques, telles que l'instabilité hémodynamique (hypotension / hypertension), la détresse respiratoire, l'insuffisance rénale aiguë, des troubles graves du rythme cardiaque, des affections neurologiques (traumatisme crânien, accident vasculaire cérébral, coma), etc. Leur association, fréquente, est appelée défaillance multi-viscérale. En effet, il est fréquent que l'une de ces défaillances en entraîne une autre, la plupart des systèmes organiques étant liés les uns aux autres.

Le séjour en service de réanimation implique un monitorage continu des fonctions vitales et, le cas échéant, le recours à des méthodes de suppléance (transfusion de dérivés sanguins, remplissage vasculaire, ventilation mécanique, catécholamines, hémodialyse, circulation extracorporelle, etc.). L'objectif final de la réanimation est la restauration de l'homéostasie.

De ce fait, les patients admis en service de réanimation risquent de contracter une infection nosocomiale, d'origine bactérienne, virale, mycosique ou parasitaire.

Une infection nosocomiale est définie comme une infection contractée à l'hôpital, c'est-à-dire qui n'est pas présente lorsque le patient est admis en milieu hospitalier, dans le cas présent en service de réanimation, et qui se déclare au minimum 48 heures après l'admission. L'infection nosocomiale peut affecter n'importe quelle partie du corps, mais les plus fréquentes en réanimation sont les pneumonies en lien avec la ventilation mécanique, les infections intra-abdominales suite à un traumatisme ou une intervention chirurgicale, les infections du tractus urinaire (ou UTI) et les bactériémies liées aux dispositifs intravasculaires (Vincent JL, Lancet, 2003 ; Eggiman P, Chest, 2001).

La prévalence des infections nosocomiales est nettement plus élevée dans les services de réanimation que celle observée dans d'autres secteurs hospitaliers, résultant de la conjonction délétère de plusieurs facteurs de risque endogènes : une exposition du patient à des procédures invasives (ventilation artificielle, sondage urinaire, cathétérisme), la gravité des patients (ainsi que les comorbidités associées) et les thérapeutiques (transfusions multiples, sédation). Néanmoins malgré l'ensemble des mesures d'hygiène et de surveillance (risques exogènes) mises en place et la prise en compte de ces facteurs de risque endogènes, l'incidence des infections nosocomiales reste stable ou diminue modestement au fil des années.

Une infection nosocomiale est susceptible de provoquer une infection disséminée, ou syndrome septique, pouvant évoluer du simple sepsis vers un sepsis sévère ou même vers un choc septique.

Ces trois syndromes cliniques, sepsis, sepsis sévère et choc septique ont été définis, par ordre croissant de gravité, en 1992 par un groupe d'experts (R.C. Bone *et al.,* 1992,) :

- Le sepsis est ainsi une réponse systémique inflammatoire liée à une infection,
- Un sepsis sévère est un sepsis accompagné du disfonctionnement d'au moins un organe,
- Le choc septique est un sepsis sévère associé à une hypotension persistante et peut être qualifié par :

   ◦ La présence d'un site infectieux identifié,
   ◦ Une réponse inflammatoire généralisée se manifestant par au moins trois des signes suivants : a) température supérieure à 38°C ou inférieure à 36°C, b) rythme cardiaque supérieur à 90 battements par minute, c) rythme respiratoire supérieur à 20 respirations par minute, d) nombre de leucocytes supérieur à $12000/mm^3$ ou inférieur à $4000/mm^3$,
   ◦ Une hypotension persistante malgré des remplissages adéquats et des traitements vasopresseurs

Identifier les patients les plus à risque de contracter une infection disséminée, dès leur admission et en l'absence de tout symptôme d'infection disséminée, permettrait de mettre en place le plus tôt possible une stratégie pour limiter leur risque de développer une telle infection. Par exemple, un traitement antibiotique en prophylaxie pourrait être administré (Puisieux F *et al.,* 1993 ; Jensen JU *et al.,* 2011) ou encore un traitement préventif (K. Asehnoune *et al.,* 2014) ou encore une immunothérapie ciblée (Chahin A *et al.,* 2015 ; Ali YM *et al.,* 2014) , ou plus simplement, les voies d'entrée pour les pathogènes pourraient être limitées (i.e. retrait des cathéters dès que possible...). Ces mesures permettraient une meilleure prise en charge du patient conduisant à :

- une réduction de la durée d'hospitalisation en réanimation et à l'hôpital ce qui permet une diminution des coûts associés (Lambert ML SC *et al.,* 2011);
- une diminution des complications septiques ; et
- une diminution du taux de mortalité.

Il existe donc un besoin urgent, non résolu depuis de nombreuses années, de trouver de bons outils pour être capable de prédire, chez les patients admis en service de réanimation, n'ayant aucun symptôme clinique d'infection disséminée, lesquels risquent le plus de développer une infection disséminée et ainsi permettre leur stratification dès leur admission, en fonction de leur risque de développer ce type d'infection.

D'une manière surprenante, les Demanderesses ont mis en évidence qu'une telle prédiction était rendue possible par l'analyse de la dose de gelsoline dans un échantillon biologique issu du patient admis en service de réanimation. En effet, les Demanderesses ont montré que les patients à haut risque de développer une telle infection disséminée voyaient leur taux de gelsoline grandement diminuer. L'invention présente donc une avancée importante dans le domaine de la lutte contre le développement d'infections disséminées, qui est l'une des premières causes de mortalité chez des patients à risque dans le cadre d'une admission en service de réanimation, lesquels patients ne présentent à leur admission aucun symptôme d'infection disséminée.

Ainsi, la présente invention a pour premier objet un procédé de prédiction du risque de développer une infection disséminée chez un patient admis en service de réanimation n'ayant aucun symptôme clinique d'une telle infection, comprenant ou consistant en les étapes suivantes :

- déterminer une première dose de gelsoline G1 dans un échantillon biologique dudit patient issu d'un premier prélèvement au temps T1, effectué entre le jour d'admission en service de réanimation et 48 heures après,
- déterminer une deuxième dose de gelsoline G2 dans un échantillon biologique dudit patient issu d'un second prélèvement au temps T2, effectué deux à trois jours après le premier prélèvement,
- calculer la variation entre la dose de gelsoline G2 et la dose de gelsoline G1, donnant une valeur Δ,
- comparer la valeur Δ obtenue à la précédente étape, à une valeur seuil S préalablement déterminée à partir de deux populations de patients admis en service de réanimation, l'une n'ayant pas développé une infection disséminée et l'autre ayant développé une telle infection,

  - une valeur Δ inférieure à ladite valeur seuil S signifiant que le patient admis en service de réanimation est un patient à haut risque de développer une infection disséminée, et

  - une valeur Δ supérieure à ladite valeur seuil S signifiant que le patient admis en service de réanimation n'est pas un patient à haut risque de développer une infection disséminée.

Les Demanderesses ont donc montré, contre toute attente, qu'il était possible de prédire le risque de développer une infection disséminée, chez un patient admis en service de réanimation, en utilisant la gelsoline en tant que marqueur.

Les patients admis en service de réanimation, selon la présente invention, sont des patients qui présentent ou sont susceptibles de présenter plusieurs défaillances viscérales aiguës mettant directement enjeu le pronostic vital et impliquant le recours à des méthodes de suppléance. Ces patients sont par exemple des patients polytraumatisés, des patients dits grand-brûlés, des patients atteints de pancréatites ou encore de syndrome respiratoire aigüe. Ces patients sont donc particulièrement vulnérables aux infections nosocomiales pouvant évoluer vers une infection disséminée.

Une infection disséminée, selon la présente invention s'entend par une infection non localisée, généralisée au sein de l'organisme et provoquée par des pathogènes tels que bactéries, champignons, virus ou parasites. On parle aussi de syndrome septique.

Par prédiction du risque de développer une infection disséminée, on entend l'identification de patients sans symptômes d'infection disséminée au moment de l'admission en service de réanimation, c'est-à-dire ne présentant aucune manifestation clinique d'une telle infection disséminée, lesquels vont la développer dans les jours suivant leur admission en service de réanimation, en moyenne 5 jours après leur admission. En d'autres termes, par prédiction du risque de développer une infection disséminée, on entend la détermination d'un tel risque chez un patient admis en réanimation, lequel n'a aucun symptôme d'infection disséminée au moment de l'admission en service de réanimation. Les patients les plus à risque de développer une infection disséminée et pour lesquels il convient de mettre en place le plus tôt possible une stratégie pour limiter leur risque de développer une telle infection sont dits patients à haut risque. Pour ces patients à haut risque, l'utilisation de la gelsoline permet de prédire la survenue d'une infection disséminée. En d'autres termes, un patient à haut risque est un patient dont la probabilité de développer une infection disséminée est au moins de 75%.

Dans le cadre de la présente invention, l'infection est dite précoce c'est-à-dire qu'elle va survenir dans les 5 jours qui suivent l'admission des patients en service de réanimation.

Le marqueur utilisé dans la présente invention pour la prédiction du risque de développer une infection disséminée est la gelsoline. C'est une protéine qui a un poids moléculaire entre 82 et 84 kDa (N° Swiss Prot P06396), étant cytosolique et comprenant 6 domaines (G1 à G6) capables de se lier aux monomères d'actine ou aux filaments d'actine en présence d'une forte concentration en ions calcium. Cette liaison peut être régulée par le pH, les phosphoisnositides, l'acide lisophosphatidique et des concentrations élevées en calcium. Les sites de liaison aux monomères d'actine sont présents dans le domaine G1 ainsi que dans le segment G4-6 tandis que le site de liaison de forte affinité avec les filaments d'actine se trouve dans le segment G2-3. La gelsoline joue alors un rôle de réparation dans les dommages tissulaires. Il existe 4 isoformes connues de la protéine dont la principale est l'isoforme 1 (N°Swiss Prot P06396-1) qui est la forme plasmatique, sécrétée de la protéine. L'isoforme 2 (N°Swiss Prot P06396-2), cytoplasmique diffère de l'isoforme 1 par la délétion des 51 premiers acides aminés. Les isoformes 3 et 4 (N°Swiss Prot P06396-3 et P06396-4), cytoplasmiques, diffèrent de l'isoforme 1 par une modification de la séquence des 48 premiers acides aminés.

Dans le cadre de la présente invention, toutes les isoformes de la gelsoline peuvent être utilisées en tant que marqueur pour prédire le risque, chez un patient admis en service de réanimation, de développer une infection disséminée. Selon un mode de réalisation, l'isoforme dosée est l'isoforme 1.

La gelsoline est connue pour être impliquée dans de nombreuses pathologies, notamment dans différents types de cancers, dans certains contextes inflammatoires et infectieux, dans le cas des maladies cardiaques et pulmonaires, dans la maladie d'Alzheimer ou encore le vieillissement.

Cette protéine a déjà été décrite en tant que marqueur dans la littérature scientifique :

- soit pour le diagnostic d'un syndrome septique, c'est-à-dire l'identification de patients déjà atteints par un syndrome septique et ayant des symptômes cliniques d'infection (Lee *et al.*,2008), en faisant un lien avec la sévérité et la prédiction de mortalité (Xianhui et al., 2014),
- soit pour le pronostic de l'évolution du syndrome septique chez des patients septiques, c'est-à-dire l'estimation de cette évolution négative chez des patients déjà reconnus comme ayant une infection disséminée (Wang *et al.,* 2008),
- soit pour la prédiction de mortalité chez des patients septiques, c'est-à-dire l'estimation du risque de mortalité chez des patients déjà reconnus comme ayant une infection disséminée (Wang *et al.,* 2008),

Mais en aucun cas la gelsoline n'a été décrite pour la prédiction du risque de développer une infection disséminée chez des patients à risque, vulnérables, tels que des patients admis en service de réanimation, pour lesquels aucune manifestation clinique d'infection disséminée n'est présente.

La prédiction du risque de développer une infection disséminée chez les patients admis en service de réanimation est mise en oeuvre par la détermination d'une dose de gelsoline dans un échantillon biologique dudit patient.

D'une manière générale, le terme « échantillon » se réfère à une partie ou à une quantité, plus particulièrement une petite partie ou une petite quantité, prélevée à partir d'une ou plusieurs entités aux fins d'analyse. Cet échantillon peut éventuellement avoir subi un traitement préalable, impliquant par exemple des étapes de mélange et de dilution.

L'échantillon dans le cadre du procédé de l'invention est un échantillon biologique provenant du patient chez qui on souhaite déterminer le risque de développer une infection disséminée. En particulier, un tel échantillon biologique est choisi parmi ceux susceptibles de contenir la gelsoline.

L'échantillon biologique selon la présente invention peut être de différentes natures. En particulier, cet échantillon est un fluide biologique, par exemple choisi parmi le sang, le sang total (tel que collecté de la voie veineuse, c'est-à-dire contenant les cellules blanches et rouges, les plaquettes et le plasma), le sérum, le plasma, le liquide de lavage broncho-alvéolaire, le liquide céphalo-rachidien, également appelé liquide cérébro-spinal, et les urines. De préférence, l'échantillon biologique issu du patient est un échantillon de sang total, plasma, sérum ou tout dérivé.

Par « dose de gelsoline », on entend une quantité de gelsoline dans ledit échantillon biologique. Parfois, ce n'est pas la dose qui est donnée comme résultat final du procédé mais la concentration en gelsoline, laquelle est calculée à partir de la dose en divisant la dose par le volume de l'échantillon sur lequel est pratiquée la mesure. Aux fins de l'invention, on appellera indifféremment « dose » la dose ou la concentration en gelsoline.

La détermination de la dose de gelsoline dans un échantillon biologique peut se faire selon les techniques largement connues de l'homme du métier pour déterminer la quantité, ou dose, d'un analyte dans un échantillon biologique. A titre d'exemples, on peut citer les dosages par immunoessais, tels qu'ELISA (Enzyme Linked Immuno Sorbent Assay), ELFA (Enzyme Linked Fluorescent Assay) et RIA (Radio Immuno Assay), et les dosages par spectrométrie de masse, ce qui constitue un mode de réalisation de l'invention.

Le dosage par immunoessai est une méthode bien connue de l'homme du métier et largement utilisée dans le domaine de l'analyse d'échantillons biologiques. Il permet de détecter des analytes dans des échantillons sous forme notamment de protéines (antigènes/anticorps), de peptides et d'haptènes, comme par exemple les stéroïdes ou les vitamines, impliquant des réactions immunologiques entre l'analyte à détecter, dans le cas présent la gelsoline, et un ou des partenaire(s) de liaison à cet analyte. Ces méthodes d'immunoessai sont basées sur des mesures permettant de quantifier les signaux émis au cours de l'analyse de l'échantillon biologique. La quantité de signaux détectée est généralement proportionnelle à la quantité, ou dose, d'analyte à mesurer (par exemple lors d'un dosage en sandwich) ou inversement proportionnelle à la quantité, ou dose, d'analyte à mesurer (par exemple dosage en compétition). Bien entendu, le terme « immuno » dans « immunoessai » par exemple, n'est pas à considérer dans la présente demande comme indiquant strictement que le partenaire de liaison est un partenaire immunologique, tel qu'un anticorps. En effet, l'homme du métier utilise également largement ce terme lorsque le partenaire de liaison, aussi appelé ligand, n'est pas un partenaire immunologique mais est par exemple un récepteur à l'analyte que l'on veut doser. Ainsi, il est connu de parler du dosage ELISA (« Enzyme-Linked Immunosorbent Assay » littéralement « dosage d'immunoadsorption par enzyme liée ») pour des dosages qui utilisent des partenaires de liaison non immunologiques, appelés plus largement en anglais « Ligand Binding Assay », que l'on pourrait traduire par « Dosage utilisant la liaison à un ligand », alors que le terme « immuno » est inclus dans l'acronyme ELISA. Par souci de clarté, les Demanderesses utiliseront dans toute la demande le terme « immuno » pour tout dosage utilisant un partenaire de liaison, même quand il n'est pas un partenaire immunologique.

A titre de partenaire de liaison à la gelsoline, on peut citer les anticorps, les fractions d'anticorps, les nanofitines, les aptamères (Ochsner U.A. et al., 2014) ou toute autre molécule qui est connue pour avoir une interaction avec la gelsoline à rechercher, telle que les lipopolysaccharides (Bucki R. et al., 2005)

Les anticorps partenaires de liaison sont par exemple soit des anticorps polyclonaux, soit des anticorps monoclonaux dont l'obtention est largement connue de l'homme du métier. De tels anticorps sont disponibles dans le commerce, comme par exemple les anticorps polyclonaux suivants : Anti-Gelsolin plasma Goat polyclonal antibody, ABCAM, anti-Gelsolin polyclonal antibody SHEEP 1, Thermo Pierce ANBOP0000269032, et les anticorps monoclonaux suivants : Monoclonal anti-gelsoline 1, Boster Biological Technology ANBOP0000215379.

A titre d'exemple de fragments d'anticorps, on peut citer les fragments Fab, Fab', F(ab')2 ainsi que les chaînes scFv (Single chain variable fragment), dsFv (Double-stranded variable fragment). Ces fragments fonctionnels peuvent notamment être obtenus par génie génétique.

L'immunoessai consistant à déterminer la dose de gelsoline est un essai quantitatif largement connu de l'homme du métier mettant en oeuvre de préférence deux partenaires de liaison à la gelsoline. L'un des deux partenaires peut être couplé à un marqueur pour former un conjugué ou un traceur. L'autre partenaire de liaison peut être capturé sur un support solide. On parle alors de partenaire de capture pour ce dernier et partenaire de détection pour le premier.

Comme indiqué précédemment, le signal mesuré émis lors de l'immunoessai est alors proportionnel à la quantité, ou dose, de gelsoline dans l'échantillon biologique.

Pour corréler le signal obtenu à la dose, ou à la concentration dans l'échantillon biologique, il convient d'utiliser un modèle mathématique préétabli à partir d'une gamme étalon. Cette gamme étalon sera obtenue préalablement de façon connue. En quelques mots, l'obtention d'une gamme étalon consiste à mesurer le signal généré par des quantités ou concentrations croissantes et connues de gelsoline, à tracer la courbe donnant le signal en fonction de la dose, ou concentration, et à trouver un modèle mathématique qui représente de la manière la plus fidèle possible cette relation. Le modèle mathématique sera utilisé pour déterminer par extrapolation les doses, ou concentrations, de gelsoline inconnues, contenues dans l'échantillon biologique à tester.

Par marqueur utilisé pour former le conjugué, on entend, notamment, toute molécule contenant un groupement réactif avec un groupement du partenaire de liaison, directement sans modification chimique, ou après modification chimique pour inclure un tel groupement, laquelle molécule est capable de générer directement ou indirectement un signal détectable. Une liste non limitative de ces marqueurs de détection directe consiste en :

- les enzymes qui produisent un signal détectable par exemple par colorimétrie, fluorescence, luminescence, comme la peroxydase de raifort, la phosphatase alcaline, la β-galactosidase, la glucose-6-phosphate déshydrogénase,
- les chromophores comme les composés fluorescents, luminescents, colorants,
- les molécules radioactives comme le 32P, le 35S ou le 125I,
- les molécules fluorescentes telles que les Alexa ou les phycocyanines, et
- les sels électrochmiluminescents tels que des dérivés organo-métalliques à base d'acridinium ou de ruthénium.

Des systèmes indirects de détection peuvent aussi être utilisés, comme par exemple des ligands capables de réagir avec un anti-ligand. Le ligand correspond alors au marqueur pour constituer, avec le partenaire de liaison, le conjugué.

Les couples ligand/anti-ligand sont bien connus de l'homme du métier, ce qui est le cas par exemple des couples suivants : biotine/streptavidine, haptène/anticorps, antigène/anticorps, peptide/anticorps, sucre/lectine, polynucléotide/complémentaire du polynucléotide.

L'anti-ligand peut alors être détectable directement par les marqueurs de détection directe décrits précédemment ou être lui-même détectable par un autre couple ligand/anti-ligand, et ainsi de suite.

Ces systèmes indirects de détection peuvent conduire, dans certaines conditions, à une amplification du signal. Cette technique d'amplification du signal est bien connue de l'homme du métier, et l'on pourra se reporter aux demandes de brevet antérieures FR 2781802 ou WO 95/08000 de l'une des Demanderesses.

Selon le type de marquage utilisé, l'homme du métier ajoutera des réactifs permettant la visualisation du marquage ou l'émission d'un signal détectable par tout type d'appareil de mesure approprié, comme par exemple un spectrophotomètre, un spectrofluorimètre, un densitomètre ou encore une caméra haute définition.

L'immunoessai peut également comprendre d'autres étapes connues de l'homme du métier, telles que des étapes de lavage et des étapes d'incubation.

L'immunoessai peut être un essai en une étape ou en deux étapes, comme cela est largement connu de l'homme du métier. En quelques mots, un immunoessai en une étape comprend la mise en présence de l'échantillon à tester simultanément avec les deux partenaires de liaison, alors qu'un immunoessai en deux étapes comprend la mise en présence de l'échantillon à tester d'une part avec le premier partenaire de liaison, puis le complexe analyte-premier partenaire de liaison ainsi formé est mis en présence du deuxième partenaire de liaison.

La spectrométrie de masse, qui peut se substituer aux techniques précédemment développées que sont notamment les dosages ELISA, est une méthode largement connue de l'homme du métier. Elle est mise en oeuvre dans un spectromètre de masse. C'est un outil puissant de plus en plus utilisé pour l'analyse et la détection de différents types de molécules dans des échantillons biologiques. De façon générale, tout type de molécule pouvant être ionisé peut être détecté en fonction de sa masse moléculaire à l'aide d'un spectromètre de masse. Selon la nature de la molécule à détecter, d'origine protéique ou métabolique, certaines technologies de spectrométrie de masse peuvent être plus adaptées. Néanmoins, quelle que soit la méthode de spectrométrie de masse utilisée pour la détection, cette dernière comprend une étape d'ionisation de la molécule cible en ions dits moléculaires et une étape de séparation des ions moléculaires obtenus en fonction de leur masse. Un spectromètre de masse mesure le rapport de la masse sur la charge (m/z) de molécules ionisées qui est corrélé à la molécule cible à analyser.

Tous les spectromètres de masse comportent donc :

i) une source d'ionisation destinée à ioniser les marqueurs présents dans l'échantillon à analyser, c'est-à-dire à conférer une charge positive ou négative à ces marqueurs;

ii) un analyseur de masse destiné à séparer les marqueurs ionisés, ou ions moléculaires, en fonction de leur ratio masse sur charge (m /z) ;

iii) un détecteur destiné à mesurer le signal produit soit directement par les ions moléculaires, soit par des ions produits à partir des ions moléculaires, comme détaillés ci-après.

L'étape d'ionisation nécessaire pour la mise en oeuvre d'une spectrométrie de masse peut être mise en oeuvre par tout procédé connu de l'homme du métier. La source d'ionisation permet d'amener les molécules à doser sous un état gazeux et ionisé. Une source d'ionisation peut être utilisée soit en mode positif pour étudier les ions positifs, soit en mode négatif pour étudier les ions négatifs. Plusieurs types de sources existent et seront utilisés en fonction du résultat recherché et des molécules analysées. On peut citer, notamment :

- l'ionisation électronique (EI), l'ionisation chimique (CI) et la désorption-ionisation chimique (DCI),
- le bombardement par atomes rapides (FAB), atomes métastables (MAB) ou ions (SIMS, LSIMS),
- le couplage plasma inductif (ICP),
- l'ionisation chimique à pression atmosphérique (APCI) et la photoionisation à pression atmosphérique (APPI),
- l'électronébulisation ou électrospray (ESI),
- la désorption-ionisation laser assistée par matrice (MALDI), activée par une surface (SELDI) ou sur silicium (DIOS), et
- l'ionisation-désorption par interaction avec espèces métastables (DART).

L'analyseur de masse dans lequel est mis en oeuvre l'étape de séparation des marqueurs ionisés en fonction de leur rapport masse/charge (m/z) est tout analyseur de masse connu de l'homme du métier. On peut citer les analyseurs basse résolution, du type quadripôle ou quadrupôle (Q), piège à ions 3D (IT) ou linéaire (LIT), également appelés trappe ionique, et les analyseurs haute résolution, permettant de mesurer la masse exacte des analytes et qui utilisent notamment le secteur magnétique couplé à un secteur électrique, le temps de vol (TOF).

La séparation des ions moléculaires en fonction de leur rapport m/z peut être mise en oeuvre une seule fois (spectrométrie de masse simple ou MS), ou bien plusieurs séparations MS successives peuvent être menées. Lorsque deux séparations MS successives sont réalisées, l'analyse est appelée MS/MS ou $MS^2$. Lorsque trois séparations MS successives sont réalisées, l'analyse est appelée MS/MS/MS ou $MS^3$ et plus généralement, lorsque n séparations MS successives sont réalisées, l'analyse est appelée $MS^n$.

Le mode SRM (Selected Reaction Monitoring) d'analyse mettant en oeuvre deux séparations successives de spectrometrie de masse simple est une utilisation particulière de séparation $MS^2$.

Cette détection SRM en mode MS/MS comporte deux étapes supplémentaires par rapport au mode MS qui sont une fragmentation des ions moléculaires alors appelés ions précurseurs pour donner des ions fragments (ou ions fils) ainsi qu'une séparation des ions fragments en fonction de leur masse. C'est alors le rapport m/z des ions fragments qui est corrélé à la molécule cible à analyser. Le principe du mode SRM est de sélectionner spécifiquement un ion précurseur, de le fragmenter, puis de sélectionner spécifiquement l'un de ses ions fragments. Pour de telles applications, des dispositifs du type « triple quadripôle » ou des hybrides « triple quadripôle à trappe ionique » sont généralement utilisés.

Dans le cas d'un dispositif « triple quadripôle » ou « quadripôle à trappe ionique » (Q1q2Q3) utilisé en mode MS$^2$, en vue du dosage ou de la détection d'une protéine cible, le premier quadripôle (Q1) permet de filtrer les ions moléculaires, correspondant aux peptides protéotypiques caractéristiques de la protéine à doser et obtenus lors d'une étape antérieure de digestion, que nous détaillerons plus bas, en fonction de leur rapport masse sur charge (m/z). Seuls les peptides ayant le rapport masse/charge du peptide protéotypique recherché, rapport appelé $(m/z)_1$, sont transmis dans le deuxième quadripôle (q2) et jouent le rôle d'ions précurseurs pour la fragmentation ultérieure. L'analyseur q2 permet de fragmenter les peptides de rapport masse/charge $(m/z)_1$ en ions fragments de première génération. La fragmentation est généralement obtenue par collision des peptides précurseurs avec un gaz inerte, comme de l'azote ou de l'argon dans q2. Les ions fragments de première génération sont transmis dans un troisième quadripôle (Q3) qui filtre les ions fragments de première génération en fonction d'un rapport masse sur charge spécifique, rapport appelé $(m/z)_2$. Seuls les ions fragments de première génération ayant le rapport masse/charge d'un fragment caractéristique du peptide protéotypique recherché $(m/z)_2$ sont transmis dans le détecteur pour être détectés, voire quantifiés.

Ce mode de fonctionnement présente une double sélectivité, en relation avec la sélection de l'ion précurseur d'une part et de la sélection de l'ion fragment de première génération d'autre part. La spectrométrie de masse en mode SRM est donc avantageuse pour la quantification.

Dans le cadre de l'invention, le spectromètre de masse de type « triple quadripôle » ou « quadripôle à trappe ionique» peut-être couplé à une chromatographie choisie parmi une chromatographie d'affinité et une chromatographie liquide haute performance (HPLC). De préférence, le spectromètre de masse est couplé à un système chromatographique liquide haute performance (HPLC).

Comme indiqué précédemment, en amont de la détection par spectrométrie de masse, l'échantillon à analyser est préférentiellement traité au préalable pour générer des peptides à partir des protéines présentes dans l'échantillon, par exemple par digestion avec une enzyme protéolytique (protéase), ou par action d'un réactif chimique. En effet, le clivage des protéines peut être fait par un traitement physico-chimique, par un traitement biologique ou par une combinaison des deux traitements. Le traitement des protéines par digestion enzymatique est néanmoins préféré par rapport au traitement physico-chimique car il préserve davantage la structure des protéines, et est plus facile à contrôler.

Par « digestion enzymatique », on entend l'action simple ou combinée d'une ou de plusieurs enzymes dans des conditions de réaction appropriées. Les enzymes effectuant la protéolyse, appelées protéases, coupent les protéines à des endroits spécifiques. Dans le cadre de l'invention, la digestion de l'échantillon est, de préférence, réalisée par action d'une enzyme protéase, par exemple la trypsine qui scinde la liaison peptidique au niveau du groupe carboxylique des résidus Arginine (R) et Lysine (K).

Parmi les peptides ainsi obtenus, les peptides caractéristiques de la protéine à doser sont nommés peptides protéotypiques. Ce sont eux qui seront suivis et quantifiés par la suite en spectrométrie de masse. Le dosage quantitatif de protéines par des techniques de spectrométrie de masse via leur peptides protéotypiques a déjà été validé dans des fluides complexes (Fortin T. et al., 2009) .

Les peptides protéotypiques d'intérêt sont choisis en fonction de leur spécificité dans la matrice (grâce au logiciel Peptide Atlas disponible en accès libre sur internet) et de leur sensibilité (meilleure réponse dans le spectromètre de masse).

En fonction de la complexité de l'échantillon, l'étape de digestion pourra être suivie d'une étape de fractionnement des peptides présents dans l'échantillon d'intérêt pour réduire sa complexité. Par fractionnement, on entend, de façon classique, une épuration du nombre de peptides présents. Un tel fractionnement pourra se faire par une technique connue de l'Homme du métier comme par exemple la purification par extraction en phase solide (nommé en anglais Solide Phase Extraction ou SPE), le fractionnement « Off gel » (Michel PE. et al., 2003) ou la technique d'immobilisation de pH par gradient isoélectrique (Essader AS et al., 2005).

Un des avantages de l'utilisation de la spectrométrie de masse réside en ce qu'elle est particulièrement utile pour quantifier des molécules, dans le cas présent un marqueur protéique comme la gelsoline. Pour ce faire, on utilise l'intensité de courant détectée, laquelle est proportionnelle à la quantité de molécule cible. L'intensité de courant ainsi mesurée pourra servir de mesure quantitative permettant de déterminer la quantité de molécule cible présente. En effet, l'intensité de courant induit par le ou les ions fils sélectionnés, mesurée dans le détecteur, est proportionnelle à la quantité d'ions parents, elle-même proportionnelle à la quantité de peptides protéotypiques, elle-même proportionnelle à la quantité de la molécule d'intérêt à doser. La quantité de courant dosée, induit par les ions fils, est donc directement proportionnelle à la quantité de la molécule à doser. La sélection d'au moins une mesure quantitative associée à au moins un ion fils, et la corrélation de cette mesure quantitative à la quantité de molécule présente dans l'échantillon, permet d'obtenir un dosage quantitatif.

Comme pour l'immunoessai, un calibrage est néanmoins nécessaire. Il permet de corréler l'aire du pic mesurée, correspondant à l'intensité de courant induit par les ions détectés, à la quantité de molécule cible à doser. Pour cela, les calibrages classiquement utilisés en spectrométrie de masse pourront être mis en oeuvre, dans le cadre de l'invention. Les dosages SRM sont classiquement calibrés à l'aide de standards externes ou, de préférence, à l'aide de standards internes tels que décrits par T. Fortin et al., 2009. Dans le cas où la molécule cible est un peptide protéotypique, permettant de doser une protéine d'intérêt, la corrélation entre la mesure quantitative et la quantité de peptide protéotypique cible, et par la suite de protéine d'intérêt, est obtenue en étalonnant le signal mesuré par rapport à un signal étalon pour lequel la quantité à doser est connue. L'étalonnage peut être réalisé au moyen d'une courbe d'étalonnage, par exemple obtenue par injections successives de peptide protéotypique étalon à différentes concentrations (étalonnage externe), ou de façon préférentielle, par étalonnage interne en utilisant un peptide lourd, comme standard interne, conformément à la méthode AQUA (« absolute quantification »). Par peptides lourds on entend des peptides synthétiques dont les séquences d'acides aminés sont identiques aux peptides protéotypiques cibles sélectionnés (ions moléculaires) mais pour lesquels certains des atomes de carbone 12(12C) sont remplacés par du carbone 13(13C) et/ou les atomes d'azote 14(14N) sont remplacés par de l'azote 15(15N). En effet, ces peptides lourds ont les mêmes propriétés physico-chimiques que les peptides naturels cibles (à l'exception d'une masse plus élevée), et sont élués aux mêmes temps de rétention chromatographiques. Ces peptides constituent le standard interne (IS). Ils seront ajoutés en quantité fixe à chaque échantillon en amont du dosage en spectrométrie de masse par exemple en amont de la digestion de protéines de l'échantillon d'intérêt. Ainsi, les peptides synthétiques subissent les mêmes étapes de traitement que les protéines cibles et sont co-purifiés avec les peptides naturels cibles à doser lors du fractionnement des peptides. Ils sont injectés simultanément dans le spectromètre de masse pour le dosage .La comparaison des aires de pic des peptides naturels à doser (Analytes) avec les aires des pics des peptides lourds (Standard Interne) permet de réaliser une quantification relative de la protéine du peptide naturel cible et de remonter ainsi à la quantité relative de la protéine à doser .

La détermination de la dose de gelsoline dans un échantillon biologique peut être mise en oeuvre au moins 2 fois, à deux temps différents T1 et T2. Les échantillons biologiques prélevés à T1 et T2 sont de même nature. De préférence les échantillons biologiques prélevés à T1 et T2 sont des échantillons de sang tel que sang total, plasma, sérum ou tout dérivé de sang.

La première détermination de la dose de gelsoline, dose appelée G1, est effectuée à un temps T1 dans un échantillon biologique prélevé entre le jour d'admission en service de réanimation et 48 heures après. Selon un mode de réalisation, la première détermination de la dose de gelsoline (G1) est effectuée dans les 48 premières heures suivant l'admission. Selon un autre mode de réalisation, la première détermination de la dose de gelsoline (G1) est effectuée le jour d'admission en service de réanimation.

La deuxième détermination de la dose de gelsoline, dose appelée G2, est effectuée à un temps T2 dans un échantillon biologique prélevé deux à trois jours après le premier prélèvement, soit trois à cinq jours après le jour d'admission. Selon un mode de réalisation, la deuxième détermination de la dose de gelsoline (G2) est effectué entre 72 et 120 heures suivant l'admission.

Selon la présente invention, la méthode utilisée pour déterminer la première dose de gelsoline G1 et la deuxième dose de gelsoline G2 est la même et est telle que décrite précédemment.

Après détermination de la deuxième dose G2, le procédé de prédiction du risque de développer une infection disséminée selon l'invention comprend une étape de calcul de la variation entre la dose de gelsoline G2 et la dose de gelsoline G1, donnant une valeur Δ.

Le calcul de la valeur Δ peut être effectuée par tout calcul connu de l'homme du métier permettant de mettre en évidence une différence entre G2 et G1.

Selon un mode particulier de réalisation, la valeur Δ est calculée selon la formule suivante (I) :

$$G2 - G1 \text{ (I).}$$

Dans ce cas, la valeur Δ a la même grandeur que la dose G2 ou G1 déterminée.

Selon un autre mode particulier de réalisation, la valeur Δ correspond au taux de variation relative et est calculée selon la formule suivante (II) :

$$\frac{G2 - G1}{G1} \times 100 \text{ (II)}$$

Dans ce cas, la valeur Δ est un pourcentage.

Selon un autre mode particulier de réalisation, la valeur Δ correspond à la différence de dose par unité de temps et est calculée selon la formule suivante (III) :

$$\frac{G2-G1}{T2-T1}\ (III)$$

Dans ce cas, la valeur Δ a comme grandeur une unité de dose par unité de temps.

Le procédé de prédiction du risque de développer, pour un patient admis en service de réanimation, une infection disséminée comprend une étape de comparaison de la valeur Δ, obtenue à la précédente étape, à une valeur de référence S préalablement déterminée à partir de deux populations de patients admis en service de réanimation, l'une n'ayant pas développé une infection disséminée et l'autre ayant développé une telle infection.

Cette valeur S est toujours une valeur négative car les Demanderesses ont montré contre toute attente que la dose de gelsoline diminuait grandement entre T2 et T1 pour les patients à haut risque de développer une infection disséminée.

La détermination de la valeur de référence S selon l'invention se fait sur un échantillonnage de patients significatif, c'est-à-dire sur un nombre d'échantillons minimal pour obtenir des résultats statistiquement pertinents et donc représentatifs de la population étudiée.

Une telle détermination d'une valeur de référence S est largement connue de l'homme du métier. Elle consiste notamment à mettre en oeuvre une méthode de dosage identique à celle mise en oeuvre dans le procédé de l'invention, dans des échantillons biologiques des deux populations étudiées, et à déterminer la valeur du test (dose) permettant de faire une discrimination entre ces deux populations.

La détermination de la valeur de test (dose) S permettant de faire une discrimination entre ces deux populations est connue et est calculée grâce à la courbe ROC (Receiver Operating Characteristic Curve). Cette courbe est un graphe obtenu en portant en abscisse la fraction de faux positifs, c'est-à-dire la Spécificité comme définie ci-après, et en ordonnée la fraction de vrais positifs, c'est-à-dire la Sensibilité comme définie ci-après, pour différentes valeurs de seuil fixées. Elle représente tous les couples Sensibilité/Spécificité lorsque le seuil de décision varie sur l'étendue des valeurs observées du test. Une façon globale de quantifier l'efficacité diagnostique d'un test est d'exprimer sa performance par l'aire sous la courbe R.O.C. Par convention, cette aire est toujours ≥ 0,5. Les valeurs de l'aire sous la courbe R.O.C. varient entre 0,5 (pas de différence de distribution des valeurs de dosage entre les deux sous-groupes ; la courbe R.O.C. correspond à la bissectrice) et 1 (parfaite séparation des valeurs de dosage des deux sous-groupes ; la courbe R.O.C. passe par le point (0, 1)). L'aire sous la courbe R.O.C. est une expression quantitative de la position de la courbe R.O.C. par rapport au point (0, 1) (Hanley, J.A. and McNeil, B.J, 1982 ; Zweig, M. H. and Campbell G., 1993).

La sensibilité représente le pourcentage de Vrais Positifs parmi la totalité des Positifs, reconnus comme tels. Elle exprime l'aptitude du test à détecter les échantillons biologiques réellement positifs, qui correspondent à la pathologie. Dans un langage « probabiliste », elle correspond à la probabilité d'observer un résultat Positif sachant l'échantillon Positif.

La spécificité représente le pourcentage de Vrais Négatifs parmi la totalité des Négatifs, reconnus comme tels. Elle exprime l'aptitude du test à ne pas diagnostiquer positifs des échantillons réellement négatifs, qui correspondent à un individu sain. Dans un langage « probabiliste », elle correspond à la probabilité d'observer un résultat Négatif sachant l'échantillon Négatif.

Pour obtenir la valeur de référence S, les conditions suivantes doivent être remplies :

- les échantillons prélevés des deux populations de patients sont de préférence issus de patients admis en service de réanimation ayant les mêmes caractéristiques ou une majorité de caractéristiques communes, notamment du même sexe et/ou d'un âge similaire ou identique et/ou de même origine ethnique, avec celles du sujet ou patient chez qui on souhaite déterminer le risque de développer une infection disséminée ;
- les échantillons utilisés pour obtenir la valeur de référence S doivent être de même nature que les échantillons prélevés chez le patient dont on veut prédire le risque de développer une infection ;
- les patients ne possèdent pas de symptôme d'infection disséminée au moment du prélèvement d'échantillon biologique et leur évolution vers une infection disséminée ou non est documentée a posteriori.

Le procédé de la présente invention permet de conclure sur le niveau de risque (élevé ou non) de développer une infection disséminée chez le patient duquel est issu l'échantillon biologique, une valeur Δ inférieure à ladite valeur seuil S signifiant que le patient admis en service de réanimation est un patient à haut risque de développer une infection disséminée, et une valeur Δ supérieure à ladite valeur seuil S signifiant que le patient admis en service de réanimation n'est pas un patient à haut risque de développer une infection disséminée.

Un patient à haut risque est tel que défini précédemment.

Selon le résultat du procédé de l'invention et la stratification du patient, s'il est considéré comme étant un patient à haut risque de développer une infection disséminée, le clinicien pourra décider d'une ou plusieurs des actions suivantes : administration d'un traitement antibiotique en prophylaxie ou encore une immunothérapie ciblée, ou plus simplement, les voies d'entrée pour les pathogènes pourraient être limitées (i.e. retrait des cathéters dès que possible...).

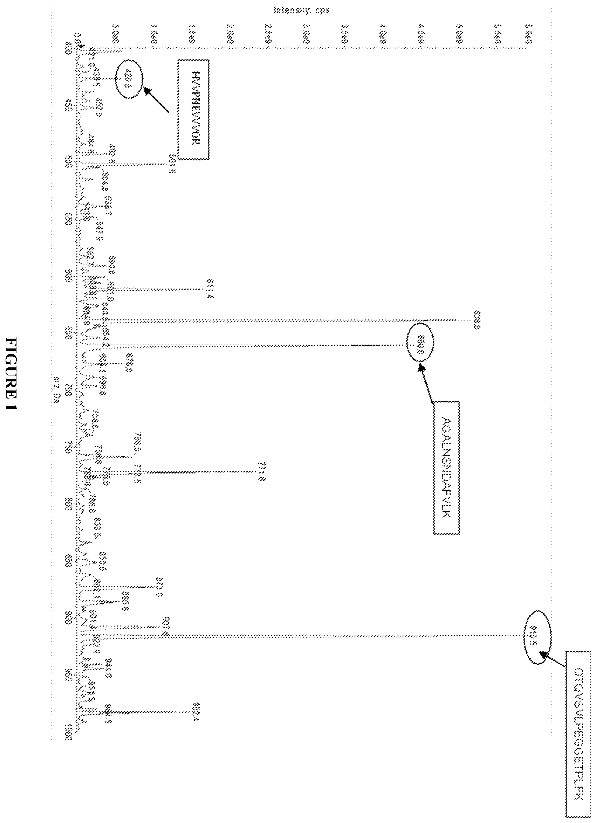

L'invention sera mieux comprise à l'aide des exemples suivants qui sont donnés à titre illustratif et non limitatif, ainsi qu'à l'aide des figures 1 à 10, dans lesquelles :

- la Figure 1 représente un graphe donnant le spectre MS en mode spectrométrie de masse amélioré EMS (Enhanced mass spectrometry) de trois peptides synthétiques protéotypiques de la protéine Gelsoline pour la sélection des ions moléculaires (ions parents), l'abscisse étant le rapport m/z en Dalton et l'ordonnée l'intensité en coups par seconde (cps) et sur lequel apparaissent les trois peptides protéotypiques utilisés.

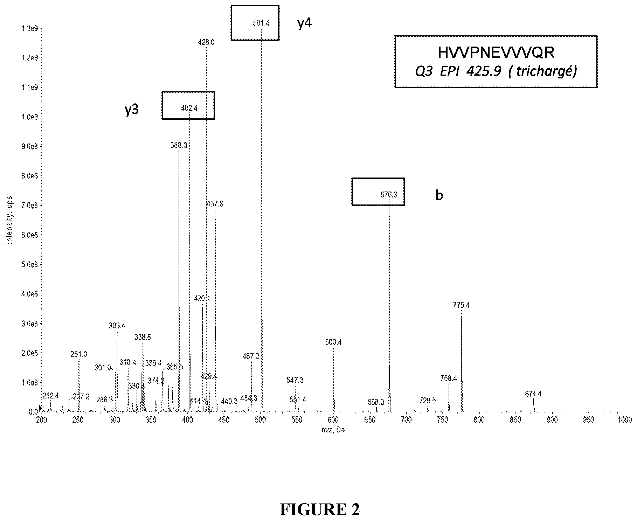

- La Figure 2 représente un graphe donnant le spectre MS en mode ion produit amélioré EPI (Enhanced product ion) pour la sélection des ions fragments obtenus à partir de l'ion parent sélectionné pour le peptide HVVPNEVVVQR, l'abscisse étant le rapport m/z en Dalton et l'ordonnée l'intensité en coups par seconde (cps).

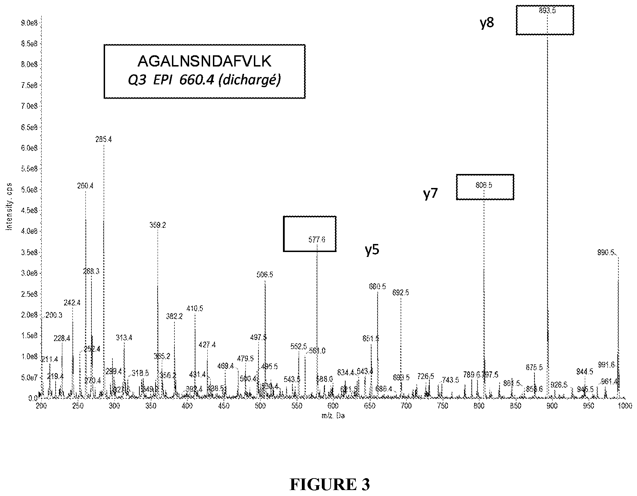

- La figure 3 représente un graphe donnant le spectre MS en mode ion produit amélioré EPI (Enhanced product ion) pour la sélection des ions fragments obtenus à partir de l'ion parent sélectionné pour le peptide AGALNSNDAFVLK, l'abscisse étant le rapport m/z en Dalton et l'ordonnée l'intensité en coups par seconde (cps).

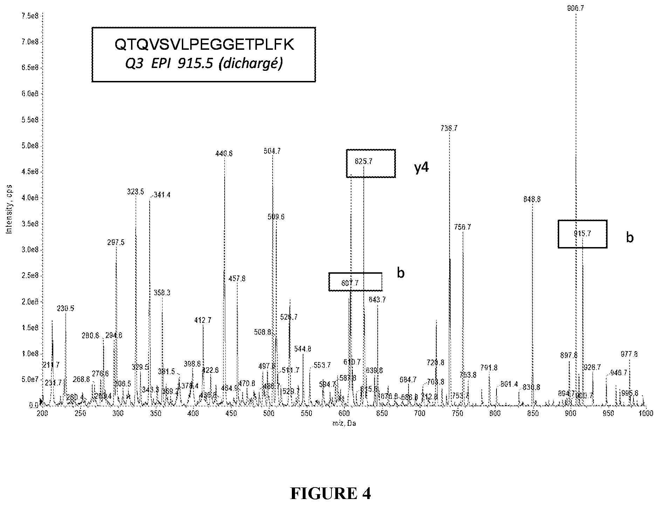

- La figure 4 représente un graphe donnant le spectre MS en mode ion produit amélioré EPI (Enhanced product ion) pour la sélection des ions fragments obtenus à partir de l'ion parent sélectionné pour le peptide QTQVSVLPEGGETPLFK, l'abscisse étant le rapport m/z en Dalton et l'ordonnée l'intensité en coups par seconde (cps).

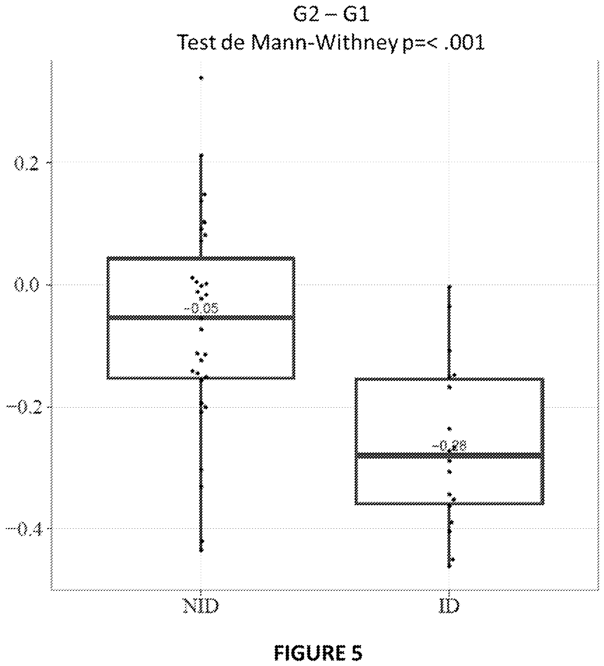

- la Figure 5 est une représentation graphique donnant, en ordonnées, la différence G2-G1 en unité de dose, en fonction d'un groupe de 49 patients admis en service de réanimation, patients dont le suivi clinique a montré qu'ils ont ensuite développé une infection disséminée (ID) et patients dont le suivi clinique a montré qu'ils n'ont pas développé ensuite une telle infection (NID) ;

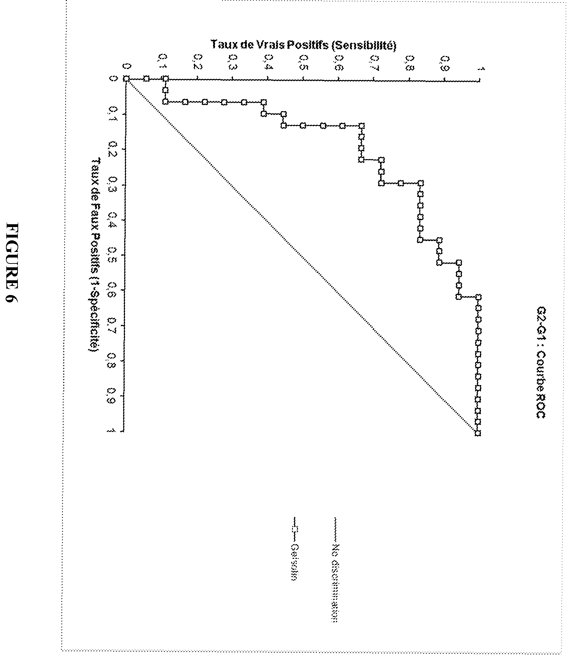

- la Figure 6 est une courbe ROC (Receiving Operating Characteristics) de la différence G2 - G1 permettant de choisir la valeur du seuil à partir des résultats de la figure 5, pour atteindre une sensibilité minimale de 75% ;

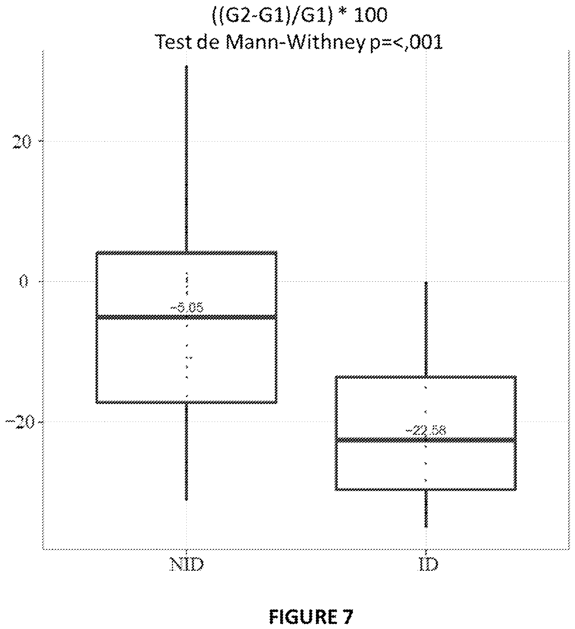

- la Figure 7 est une représentation graphique donnant, en ordonnée le taux de variation $\frac{G2-G1}{G1} \times 100$ en fonction du statut du groupe de 49 patients admis en service de réanimation, patients dont le suivi clinique a montré qu'ils ont ensuite développé une infection disséminée (ID) et patients dont le suivi clinique a montré qu'ils n'ont pas développé ensuite une telle infection (NID) ;

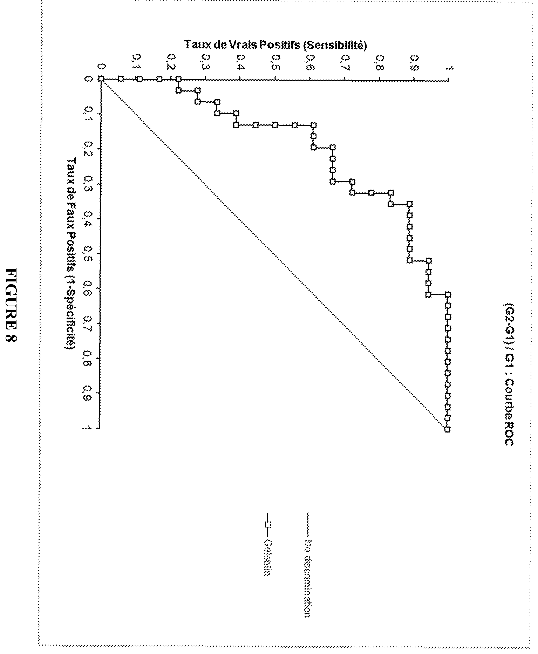

- la Figure 8 une courbe ROC (Receiving Operating Characteristics) du taux de variation $\frac{G2-G1}{G1} \times 100$, permettant de choisir la valeur du seuil à partir des résultats de la figure 7, pour atteindre une sensibilité minimale de 75%.

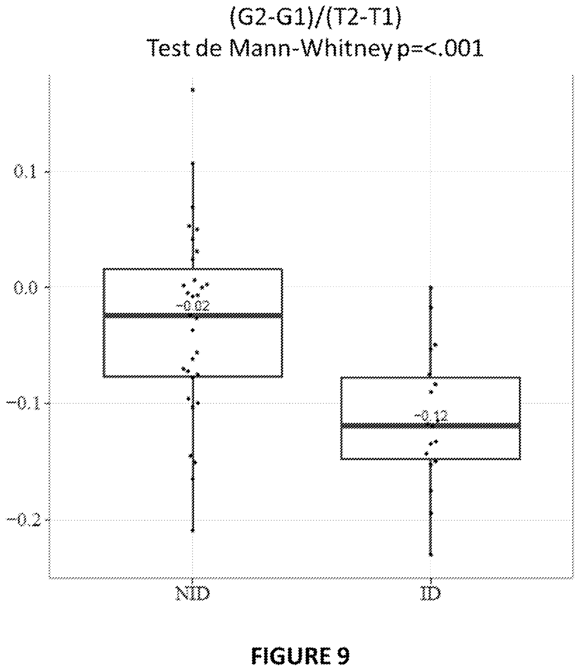

- la Figure 9 est une représentation graphique donnant, en ordonnées la différence de dose de gelsoline entre T2 et T1 corrigée par l'intervalle de temps entre les deux prélèvements, selon l'équation suivante $\frac{G2-G1}{T2-T1}$ en unité de dose/par unité de temps, en fonction du statut du groupe de 49 patients admis en service de réanimation, patients dont le suivi clinique a montré qu'ils ont ensuite développé une infection disséminée (ID) et patients dont le suivi clinique a montré qu'ils n'ont pas développé ensuite une telle infection (NID) ;

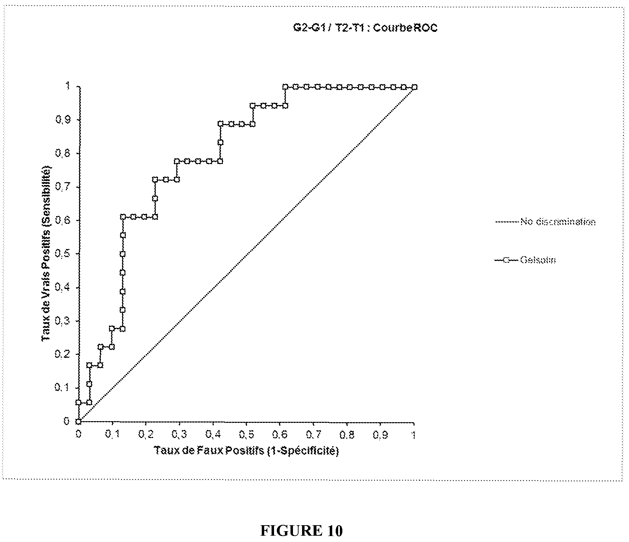

- la Figure 10 est une courbe ROC (Receiving Operating Characteristics) de la différence de dose par unité de temps calculée selon l'équation suivante $\frac{G2-G1}{T2-T1}$ permettant de choisir la valeur du seuil à partir des résultats de la figure 9, pour atteindre une sensibilité minimale de 75%.

## EXEMPLES

### Exemple 1 : Obtention et préparation des échantillons sanguins

Cette étude observationnelle rétrospective a été conduite de 2009 à 2012 chez des patients polytraumatisés sévères admis au sein du service de réanimation de l'Hôpital Edouard-Herriot de Lyon. Les critères d'inclusion étaient les suivants :

- Patients âgés de 18 ans ou plus
- ISS (Injury Severity Score) supérieur ou égal à 25
- Durée de séjour en réanimation estimée à au moins 3 jours
- Sous ventilation mécanique

Les critères cliniques d'exclusion étaient une pneumonie d'aspiration, une perforation intestinale durant le traumatisme, une thérapie immunosuppressive, et le décès durant les 48 premières heures.

49 patients polytraumatisés sévères ont été inclus dans l'étude. Tous ces patients remplissaient les critères suivants :

- le premier prélèvement sanguin a été réalisé dans les 48 premières heures suivant le traumatisme (T1) ;
- le second prélèvement sanguin a été réalisé 2 à 3 jours après le premier (96 à 120 heures après le traumatisme - T2) ;
- la date de survenue de la primo-infection, pour les patients concernés, était postérieure à la date du second prélèvement.

Parmi ces patients, 18 ont développé une infection disséminée (syndrome septique), en moyenne 5 jours après leur admission au sein du service de réanimation.

Deux prélèvements de sang sur tube EDTA ont été réalisés pour chaque patient inclus dans l'étude selon les recommandations du fournisseur. Les tubes ont ensuite été agités délicatement par retournement pendant 15 minutes avant d'être centrifugés pendant 15 minutes à 2750g et à 15°C. Les surnageants ont été prélevés délicatement puis congelés à -80°C jusqu'au dosage de la gelsoline.

## Exemple 2 : Détection de la Gelsoline par la technique LC-MS

Les étapes successives du procédé d'analyse SRM sont :

1) la digestion enzymatique
2) le fractionnement SPE (solid-phase extraction) des peptides,
3) la chromatographie liquide (LC) couplée à la MS.

Néanmoins, avant de mettre en oeuvre cette méthode, les différents couples ions parents/ions de fragmentation, ou transitions SRM, ont été identifiés comme suit :

A partir de la séquence complète de l'isoforme 1 de la gelsoline secrétée (SEQ ID N°1) obtenue dans Uniprot (N° Swissprot P06396-1). La liste des transitions SRM théoriques des 3 peptides protéotypiques sélectionnés de séquences SEQ ID N°2 à 4 pour le dosage du marqueur gelsoline a été générée en utilisant le logiciel Skyline (Brendan MacLean et al, 2010). Ce logiciel permet de réaliser une digestion trypsique fictive de la protéine à partir de sa séquence peptidique afin de générer une liste théorique de peptides.A partir de cette liste de peptides , les transitions SRM de tous les ions parents di- ou tri- chargés des peptides trypsiques théoriques dans un intervalle de masse allant de 400 à 1000 Da et tous les ions fragments possibles de type y ou b ont été prédits.

SEQ ID N°1 : isoforme 1 de la gelsoline

MAPHRPAPALLCALSLALCALSLPVRAATASRGASQAGAPQGRVPEARPNSM

VVEHPEFLKAGKEPGLQIWRVEKFDLVPVPTNLYGDFFTGDAYVILKTVQLRN

GNLQYDLHYWLGNECSQDESGAAAIFTVQLDDYLNGRAVQHREVQGFESATF
LGYFKSGLKYKKGGVASGFKHVVPNEVVVQRLFQVKGRRVVRATEVPVSWE
SFNNGDCFILDLGNNIHQWCGSNSNRYERLKATQVSKGIRDNERSGRARVHVS
EEGTEPEAMLQVLGPPALPAGTEDTAKEDAANRKLAKLYKVSNGAGTMSVSL
VADENPFAQGALKSEDCFILDHGKDGKIFVWKGKQANTEERKAALKTASDFIT
KMDYPKQTQVSVLPEGGETPLFKQFFKNWRDPDQTDGLGLSYLSSHIANVERV
PFDAATLHTSTAMAAQHGMDDDGTGQKQIWRIEGSNKVPVDPATYGQFYGG
DSYIILYNYRHGGRQGQIIYNWQGAQSTQDEVAASAILTAQLDEELGGTPVQSR
VVQGKEPAHLMSLFGGKPMIIYKGGTSREGGQTAPASTRLFQVRANSAGATRA
VEVLPKAGALNSNDAFVLKTPSAAYLWVGTGASEAEKTGAQELLRVLRAQPV
QVAEGSEPDGFWEALGGKAAYRTSPRLKDKKMDAHPPRLFACSNKIGRFVIEE
VPGELMQEDLATDDVMLLDTWDQVFVWVGKDSQEEEKTEALTSAKRYIETDP
ANRDRRTPITVVKQGFEPPSFVGWFLGWDDDYWSVDPLDRAMALAA

Les peptides protéotypiques sélectionnés pour la Gelsoline à partir de la séquence SEQ ID N°1 sont HVVPNEVVVQR (SEQ ID N°2), AGALNSNDAFVLK (SEQ ID N°3), QTQVSVLPEGGETPLFK (SEQ ID N°4).

Les 3 peptides protéotypiques sélectionnés ont ensuite été synthétisés chimiquement et infusés directement dans le spectromètre de masse afin de sélectionner les transitions les plus intenses et d'optimiser, pour chacune d'entre elles, les paramètres du spectromètre :

- L'énergie de collision (CE)
- la tension d'orifice (DP, declustering potential)
- le potentiel renforcé (EP enhanced potentiel) et
- la tension à la sortie de la cellule de collision (CXP, collision cell exit potential).

Les spectres de masse en mode spectrométrie de masse amélioré EMS (Enhanced mass spectrometry) pour la sélection des ions parents sont représentés sur la Figure 1 et ceux en mode ion produit amélioré EPI (Enhanced product ion) pour la sélection des ions fragmentés sélectionnés à partir des ions parents HVVPNEVVVQR, AGALNSNDAFVLK et QTQVSVLPEGGETPLFK sont représentés respectivement sur les Figures 2 à 4.

Le résultat de cette sélection est récapitulé dans le Tableau 1.

Tableau 1

| Séquences (SEQ ID N°) | pI | TR | Fraction d'élution | Q1 (peptide léger naturel) | Q3 (peptide léger naturel) | Q1 (peptide lourd standard interne) | Q3 (peptide lourd standard interne) | DP | EP | CE | CXP |
|---|---|---|---|---|---|---|---|---|---|---|---|
| **HVVPNEVVVQR** (SEQ ID N°2) | 5.9 | 10,1 | F5-8 | 425,9 | 402,2<br>501,3<br>676,3 | 429,2 | 412,2<br>511,3<br>676,3 | 30<br>30<br>30 | 11<br>11<br>11 | 21<br>19<br>17 | 11<br>12<br>16 |
| **AGALNSNDAFVLK** (SEQ ID N°3) | 5.88 | 15,5 | F5 | 660,4 | 577,4<br>806,5<br>893,5 | 664,4 | 585,4<br>814,5<br>901,5 | 64<br>64<br>64 | 12<br>12<br>12 | 40<br>32<br>32 | 14<br>19<br>21 |
| **QTQVSVLPEGGETPLFK** (SEQ ID N°4) | 4.53 | 21,3 | F5 | 915,5 | 457,2<br>504,3<br>756,4 | 919,5 | 457,2<br>512,3<br>756,4 | 31<br>31<br>31 | 3,5<br>3,5<br>3,5 | 40<br>57<br>37 | 12<br>12,5<br>18 |

1) Digestion enzymatique :

Les échantillons de plasma (volume 100 µl surchargés avec 20 µl de peptides lourds synthétiques), obtenus dans l'exemple 1, sont dénaturés et réduits dans une solution d'urée 6M tamponnée par du bicarbonate d'ammonium (pH 8, 5 mM) et contenant 15 mM de dithiothreitol, pendant 40 minutes à 60°C, puis alkylés par de l'iodoacétamide 35 mM, à température ambiante, pendant 40 minutes, à l'obscurité. Les échantillons sont ensuite dilués 8 fois avec du tampon bicarbonate d'ammonium 50 mM pH 8 avant d'être digérés 4 heures à 37 °C avec 200 µg de trypsine (Sigma). Un deuxième cycle de réduction-alkylation est effectué avant une deuxième étape de digestion trypsique réalisée à 37°C, pendant une nuit.

2) Fractionnement SPE :

Les échantillons digérés sont soniqués aux ultrasons pendant 15 minutes et centrifugés 30 minutes à 15000 g. Ils sont ensuite chargés sur les cartouches mixtes (hydrophobes et échangeuses d'ions) Oasis MCX (mixed cation exchange) et Oasis MAX (mixed anion exchange) 60 mg (Waters). Les échantillons chargés sur les cartouches MCX sont au préalable acidifiés avec une solution à pH 3 composée d'eau / acide formique 2%. Les échantillons chargés sur les cartouches MAX sont au préalable basifiés avec une solution à pH 12 composée d'eau / hydroxyde d'ammonium 10%. Les cartouches sont équilibrées au préalable en méthanol.

La procédure de fractionnement est décrite dans le tableau 2 ci-dessous :

Tableau 2

| ETAPES | Fonction | MCX | MAX |
|---|---|---|---|
| Conditionnement | Activation de l'adsorbant | $H_2O$ /2% AF pH =3 | $H_2O$ /10% NH3 pH = 12 |
| Dépôt de l'échantillon | Fixation des peptides d'intérêt Elimination des sels | Volume = 5,5 mL | Volume = 5,5 mL |
| Lavages | Elimination des contaminants | H2O /2% AF pH =3 | H2O /10% NH3 pH = 12 |
| **Elution 1** = Fraction MCX-F5 ou MAX F8 | Elution des peptides d'intérêt Concentration de l'échantillon | Méthanol 50% /50% tampon **pH 5** (V/V) | Méthanol 50% /50% tampon **pH 8** (V/V) |
| **Elution 2** = Fraction MCX-F5-8 ou MAX F8-4 | Elution des peptides d'intérêt Concentration de l'échantillon | Méthanol 50% /50% tampon **pH 8** (V/V) | Méthanol 50% /50% tampon **pH 4** (V/V) |
| **Elution 3** = Fraction MCX-F5-8-12 ou MAX F8-4-3 | Elution des peptides d'intérêt Concentration de l'échantillon | Méthanol 90% /10% tampon **pH 12** (V/V) | Méthanol 80% /20% tampon **pH 3** (V/V) |

Les peptides à doser sont élués par 1 ml d'un mélange méthanol/tampon bicarbonate d'ammonium (v/v). Le pH du tampon bicarbonate est choisi en fonction du point isoélectrique du peptide protéotypique d'intérêt. Les éluats sont partiellement évaporés sous un flux d'azote à l'aide d'un système TurboVap® (Biotage), puis le volume est ajusté pour obtenir un volume de 250 µl à l'aide d'une solution d'eau contenant 0,5% d'acide formique.

3) Chromatographie liquide (LC) et spectrométrie de masse (MS) :

Un aliquote de 100 µl est injecté dans la LC couplée à un système MS selon les caractéristiques suivantes.

L'analyse LC-MS est effectuée sur un système chromatographique haute pression (HPLC) de type Nexera LC (Shimadzu) avec pompe binaire et injecteur couplé à un spectromètre de masse, AB Sciex 5500 QTrap (MS hybride triple quadripôle - trappe ionique) fonctionnant en mode SRM (Qlq2Q3). La séparation LC est effectuée sur une colonne C18 Phase Reverse 2.1x150 mm, 3.6 µm (Aeris Peptide, Phenomenex) à un débit d'élution de 250 µl/min. Eluent A = 0,1% acide formique dans l'eau, éluent B = 0,1%) acide formique dans méthanol. On réalise un gradient isocratique à 5% de solvant B pendant 2 min puis un gradient linéaire de 5%B à 60%B en 37 min, puis un gradient isocratique à 5% solvant B en 2 min. L'analyse MS est réalisée en mode ESI (ionisation par électrospray) d'ionisation positive à une tension de 5500 V appliquée à l'aiguille permettant l'ionisation dans la source. Les débits du gaz de nébulisation (air) et du gaz rideau (azote) sont de 40 et 50 psi, respectivement. La source ionique Turbo VTM est réglée à 550°C, le flux d'azote auxiliaire à 40 psi.

Le contrôle de l'instrument et l'acquisition des données sont réalisés avec le logiciel Analyst 1.5.2.

Une méthode d'acquisition Schedule-SRM a été construite pour chaque fraction en utilisant les paramètres spécifiques de chaque transition (DP,EP,CE,CXP et temps de rétention). Une fenêtre de 4 min autour du temps de rétention de chaque peptide observé lors des essais avec les échantillons de sérum et plasma surchargés en peptides synthétiques ainsi qu'un « target scan time » de 1,15 seconde ont été utilisés pour toutes les acquisitions de données.

Le retraitement des données brutes SRM a été réalisé à l'aide du logiciel Multiquant 2.1 (AB Sciex) et l'algorithme d'intégration Signal Finder. Le logiciel permet l'extraction d'un chromatogramme correspondant à chaque peptide.

Pour le calibrage, des peptides lourds synthétiques de séquences identiques aux peptides cibles sélectionnés (ions fils) ont été synthétisés, avec de la lysine ou de l'arginine marquées (C13 et N15) : +8 Dalton pour la lysine, +10 Dalton pour l'arginine, afin de pouvoir être utilisés comme standard internes. Les échantillons ont été surchargés avec ces peptides lourds marqués.

Pour toutes les transitions sélectionnées (tableau 2) correspondant aux peptides de SEQ ID N°:2, SEQ ID N°:3, SEQ ID N°:4, le ratio des aires du pic chromatographique de la transition naturelle divisée par l'aire de la transition lourde a été déterminé pour obtenir une dose relative par transition pour chaque échantillon. Les doses relatives de chaque transition d'un même échantillon ont ensuite été compilées entre elles pour obtenir une dose unique, représentative de la concentration de la protéine dans l'échantillon biologique étudié. Par compilées, on entend combinées entre elles plusieurs doses indépendants mais corrélées de la même protéine pour obtenir une dose unique par protéine. Cette compilation a été réalisée en faisant la médiane des ratios.

### Exemple 3 : Analyse statistique des données

Les patients ont été divisés en 2 groupes :

- ∘ Les patients dont le statut clinique a montré qu'ils n'ont pas développé une infection disséminée (« NID ») : N=31
- ∘ Les patients dont le statut clinique a montré qu'ils ont développé une infection disséminée (« ID ») : N=18

Une analyse de régression logistique univariée et multivariée a été réalisée pour identifier les variables associées au risque d'infection en déterminant les Odd Ratios (ORs) et les intervalles de confiance associés (95% CIs).

La courbe ROC (Receiver Operating Characteristic) et les aires sous la courbe (AUC : Area Under Curve) ont été calculées pour les valeurs Δ G2-G1, $\frac{G2-G1}{G1} \times 100$ et $\frac{G2-G1}{T2-T1}$. L'analyse statistique des données a été réalisée en utilisant le langage R-3.0.0.

### Résultats

### Association entre la dose de gelsoline plasmatique chez des patients admis en service de réanimation et la survenue d'une infection disséminée.

L'association de la variable gelsoline et d'autres variables cliniques avec le statut du patient (dans le cas présent « a développé une infection disséminée ») a été testée au moyen d'une régression logistique. La force de l'association a été estimée avec le calcul des Odd Ratios (ORs), qui est le ratio de la probabilité de développer une infection disséminée sur la probabilité de ne pas développer une infection disséminée.

Les variables cliniques étudiées sont : sexe, score de gravité du traumatisme (ISS : Injury Severity Score), indice de gravité simplifié (IGS II), traumatisme neurologique, traumatisme pneumologique, transfusion de globules rouges, transfusion de plasma frais congelé.

La variable dose de gelsoline a été mesurée selon la méthode décrite ci-dessus en déterminant la différence du taux de gelsoline G2 - G1 entre T2 et T1.

Pour chaque variable qualitative : traumatisme neurologique, traumatisme pneumologique, transfusion de globules rouges et transfusion de plasma frais congelé, l'Odd Ratio est interprété de la façon suivante :

- OR = 1 : pas d'association

- OR < 1 : le risque de développer une infection disséminée est plus fréquent pour les patients qui n'ont pas cette caractéristique

- OR > 1 : le risque de développer une infection disséminée est plus fréquent pour les patients qui ont cette caractéristique.

Pour chaque variable sexe, l'Odd Ratio est interprété de la façon suivante :

- OR = 1 : pas d'association

- OR < 1 : le risque de développer une infection disséminée est plus fréquent pour les femmes

- OR > 1 : le risque de développer une infection disséminée est plus fréquent pour les hommes.

Pour chaque variable quantitative : différence du taux de gelsoline G2 - G1, ISS et IGS II, l'Odd Ratio est interprété de la façon suivante :

- OR = 1 : pas d'association

- OR < 1 : une augmentation du 1er au 3ème quartile est associée à une diminution du risque de développer une infection disséminée

- OR > 1 : une augmentation du 1er au 3ème quartile est associée à une augmentation du risque de développer une infection disséminée.

L'ensemble de ces variables a été suivi sur des patients admis en service de réanimation et qui ont développé une infection disséminée dans les jours qui suivent leur admission.

Des régressions logistiques uni-variées ont été réalisées pour chacune de ces covariables. Ensuite, les variables avec une p-value inférieure à 0,1 dans l'analyse univariée ont été conservées dans l'analyse multi-variée, ce qui a conduit à déterminer les Odd Ratios ajustés.

Les résultats sont donnés dans le tableau 3 ci-après.

Tableau 3

| Variables | Analyse univariée | | | | Analyse multivariée | | | |
|---|---|---|---|---|---|---|---|---|
| | OR | 95% CI | | P-value | OR | 95% CI | | P-value |
| Sexe ( masculin) | 2.21 | 0.59 | 8.32 | 0.2408 | | | | |
| ISS (34 - 50) | 0.99 | 0.93 | 1.05 | 0.9869 | | | | |
| IGS II (33 - 58) | 2.56 | 2.46 | 2.67 | 0.0797 | 3.60 | 0.13 | 99.73 | 0.0633 |
| Neurotraumatisme (oui) | 3.17 | 0.94 | 10.70 | 0.0635 | 3.74 | 1.00 | 1.11 | 0.0893 |
| Trauma Pneumologique (oui) | 0.38 | 0.10 | 1.38 | 0.1417 | | | | |
| Transfusion globules rouges (oui) | 1.82 | 0.18 | 18.95 | 0.6158 | | | | |
| Transfusion plasma frais congelé (oui) | 1.43 | 0.37 | 5.55 | 0.6036 | | | | |
| Gelsoline G2-G1 (-0.288 - (-0.002)) | 0.11 | 0.001 | 12.33 | 0.0013 | 0.10 | 0.02 | 0.46 | 0.0021 |

Les résultats dans le tableau 3 montrent que la différence de dose de gelsoline plasmatique mesurée à deux ou trois jours d'intervalle (T2-T1) était significativement associée avec le statut du patient (« a développé une infection disséminée ») et donc avec le risque de développer une infection disséminée. En effet, les analyses de régression logistique uni-variées ont déterminé un Odd Ratio de 0,11 pour la différence de gelsoline entre T2 et T1 et de 0,1 en analyse multi-variée, avec respectivement une p-value de 0.0013 et 0,002, comme le montre le tableau 3. De plus, les autres variables ne sont pas significatives dans l'analyse multi-variée.

Un patient avec une différence de gelsoline G2-G1 à -0.288 a 10 fois plus de risque de développer une infection disséminée qu'un patient avec une différence de gelsoline G2-G1 à -0.002.

**Prédiction de la survenue d'une infection disséminée par la mesure de la dose de gelsoline plasmatique**

Au-delà de l'association entre la dose de gelsoline plasmatique et le risque de développer une infection disséminée, notre étude a montré que la différence de dose de gelsoline plasmatique mesurée dans deux prélèvements successifs, réalisés à deux ou trois jours d'intervalle, permettait de prédire la survenue d'une infection disséminée dans les jours qui suivaient (< J8).

$$\textbf{Valeur } \Delta = \textbf{G2-G1}$$

Les résultats de la différence G2-G1 pour les 49 patients sont donnés sur la Figure 5 donnant, en ordonnées, la différence G2-G1 en unité de dose, en fonction d'un groupe de 49 patients admis en service de réanimation, patients dont le suivi clinique a montré qu'ils ont ensuite développé une infection disséminée (ID) et patients dont le suivi clinique a montré qu'ils n'ont pas développé ensuite une telle infection (NID).

Les résultats sur la figure 5 montrent que la dose de gelsoline entre les deux prélèvements diminue toujours (G2-G1 < 0) pour les patients qui vont développer une infection disséminée dans les jours qui suivent et que cette diminution est nettement plus marquée dans cette population, par rapport aux patients qui ne vont pas développer une infection disséminée.

La détermination d'une valeur seuil pour permettre la prédiction entre les patients à haut risque de développer une infection disséminée et les patients qui ne sont pas à haut risque, à partir de la valeur Δ obtenue à l'aide de l'équation G2-G1, a été effectuée au moyen de la courbe ROC telle que donnée sur la Figure 6. Ainsi, en prenant une valeur seuil fixée à - 0.144839402777984 (valeur négative), il est possible de prédire chez les patients ayant une valeur Δ inférieure à cette valeur seuil la survenue d'une infection dans les jours qui suivent, avec une sensibilité de 83,3% et une spécificité de 71%.

$$\textbf{Valeur } \Delta = \frac{G2-G1}{G1} \times \textbf{100}$$

On a également exprimé le résultat en variabilité relative par le calcul du taux de variation, selon l'équation (II), comme suit

$$\frac{G2-G1}{G1} \times 100.$$

Les résultats sont donnés sur la Figure 7 sur laquelle est indiquée la valeur médiane chez les patients qui vont faire l'objet d'une infection disséminée, cette valeur médiane est de -22% (la dose de gelsoline diminue de 22% (en médiane) entre T1 et T2 chez ces patients, contre 5,05% chez les patients qui ne vont pas faire une infection).

La détermination d'une valeur seuil avec cette cohorte lorsque la valeur Δ pour l'équation $\frac{G2-G1}{G1} \times 100$ a été effectuée au moyen de la courbe ROC, comme indiqué sur la Figure 8. Ainsi, en prenant une valeur seuil fixée égale à -0.121558271983808), il est possible de prédire chez les patients ayant une valeur Δ inférieure à cette valeur seuil la survenue d'une infection disséminée dans les jours qui suivaient avec une sensibilité de 83,3% et une spécificité de 67,7%.

Nous pouvons en conclure qu'avec ce jeu de données, cette méthode n'est pas la plus appropriée mais que cela ne limite en rien l'utilisation d'une telle méthode avec une cohorte de patients plus large.

$$\textbf{Valeur } \Delta = \frac{G2-G1}{T2-T1}$$

D'autre part, afin de prendre en compte l'intervalle de temps entre les deux prélèvements, qui n'est pas toujours homogène d'un patient à l'autre, nous nous sommes intéressés à la variation du taux de gelsoline par jour en calculant le ratio de la différence de gelsoline sur l'intervalle de temps entre les 2 prélèvements. Ce calcul a consisté à calculer le ratio de la différence de gelsoline sur l'intervalle de temps entre les deux prélèvements selon l'équation (III) ; comme suit :

$$\frac{G2-G1}{T2-T1}$$

Les résultats sont représentés sur la Figure 9. Avec ce calcul, nous avons montré que la différence médiane de la dose de gelsoline par jour est de -0.02 chez les patients qui ne vont pas développer d'infection disséminée, tandis qu'elle est de -0.12 chez les patients qui vont développer une infection disséminée.

La détermination d'une valeur seuil lorsque la valeur Δ pour l'équation $\frac{G2-G1}{T2-T1}$ a été effectuée au moyen de la courbe ROC, comme indiqué sur la Figure 10. Ainsi, en prenant une valeur seuil fixée égale à -0.072419701388992 permettait de prédire la survenue d'une infection disséminée dans les jours qui suivaient avec une sensibilité de 77,8% et une spécificité de 71%.

Nous pouvons en conclure qu'avec ce jeu de données, cette méthode n'est pas la plus appropriée mais que cela ne limite en rien l'utilisation d'une telle méthode avec une cohorte de patients plus large.

Selon les données avec les 49 patients, c'est la première méthode (Valeur Δ = G2-G1) qui permet d'avoir le meilleur compromis. Néanmoins les autres méthodes de calcul de la valeur Δ restent adaptées pour d'autres données avec une cohorte plus grande.

De manière surprenante, notre étude a montré que la mesure du taux de gelsoline plasmatique dans deux prélèvements successifs, réalisés dans les 48 premières heures suivant l'admission pour le premier puis deux à trois jours après pour le second, permettait de prédire, en absence de signes cliniques traduisant une infection disséminée, la survenue d'une infection disséminée chez des patients admis en service de réanimation.

Références bibliographiques

- Ali YM et al., 2014, Low-dose recombinant properdin provides substantial protection against Streptococcus pneumonia and Neisseria meningitidis infection, Proc Natl Acad Sci U.S.A, 111(14):5301-6

- Asehnoune K et al., 2014, Hydrocortisone and fludrocortisone for prevention of hospital-acquired pneumonia in patients with severe traumatic brain injury (Corti-TC): a double-blind, multicenter phase 3, randomized placebo-controlled trial, Lancet Respir Med, 2:706-16

- Bone R.C. et al., 1992, the ACCP/SCCM Consensus Conference Committee. American College of Chest Physicians/Society of Critical Care Medicine, Chest, 101 (6):1644-1655

- Brendan MacLean et al., 2010, BIOINFORMATICS , Vol. 26 no. 7 : 966-968

- Bucki R. et al., 2005, Inactivation of Endotoxin by Human Plasma Gelsolin, Biochemistry, 44 (28)

- Chahin A et al., 2015, The novel immunotherapeutic oligodeoxynucleotide IMT504 protects neutropenic animals from fatal Pseudomonas aeruginosa bacteriemia and sepsis, Antimicrob Agents Chemother, 59(2):1225-9

- Eggiman P et al., 2001, Infection control in the ICU, Chest, 120(6) :2059-93

- Essader AS, et al., 2005, A comparison of immobilized pH gradient isoelectric focusing and strong-cation-exchange chromatography as a first dimension in shotgun, Proteomics, 5, 24-34

- Fortin T. et al., 2009, Clinical Quantitation of Prostate-specific Antigen Biomarker in the Low Nanogram/Milliliter Range by Conventional Bore Liquid Chromatography-Tandem Mass Spectrometry (Multiple Reaction Monitoring) Coupling and Correlation with ELISA Tests. Mol. Cell Proteomics, 8(5) : 1006-1015.

- Hanley, J.A. and McNeil, B.J., 1982, The meaning and use of the area under a receiver operating characteristic (ROC) curve, Radiology, 143 : 29-36.

- Jensen JU et al., 2011, Procalcitonin-guided interventions against infections to increase early appropriate antibiotics and improve survival in the intensive care unit: a randomized trial, Crit Care Med, 39(9):2048-58

- Lambert ML SC et al., 2011, Clinical outcomes of health-care-associated infections and antimicrobial resistance in patients admitted to european intensive-care units : a cohort study, Lancet Infect Dis, 11:30-8

- Lee PS, et al., 2008, Plasma gelsolin depletion and circulating actin in sepsis: a pilot study. PLoS One. 2008; 3(11):e3712.

- Li GH, et al., 2012, Multifunctional roles of gelsolin in health and diseases. Med Res Rev. 2012 Sep;32(5):999-1025

- Michel PE, et al., 2003, Protein fractionation in a multicompartment device using Off-Gel, isoelectric focusing, Electrophoresis, 24, 3-11

- Oschsner U.A. et al., 2014, Systematic selection of modified aptamer pairs for diagnostic sandwich assays, BioTechniques, 56: 125-133

- Puisieux F, et al., 1993, Prophylactic antibiotherapy using cefapirin in the surgery of duodenal ulcer : a randomized clinical trial, Ann Fr Anesth Reanim,, 12(3) :289-92

- Vincent JL, 2003, Nosocomial infections in adult intensive-care units, Lancet, 361(9374):2068-77

- Wang H, et al., 2008, Time course of plasma gelsolin concentrations during severe sepsis in critically ill surgical patients. Crit Care. 2008; 12(4) :R106.

- Xianhui L. et al., 2014, The association between plasma gelsolin level and prognosis of burn patients. Burns, vol.40, no. 8, pages 1552-1555

- Zweig, M. H. and Campbell G., 1993, Receiver-Operating Characteristic (ROC) Plots : a fundamental evaluation tool in clinical medicine, Clin. Chem., 39/4 : 561-577

**Revendications**

1. Procédé de prédiction du risque de développer une infection disséminée chez un patient admis en service de réanimation n'ayant aucun symptôme clinique d'une telle infection, comprenant les étapes suivantes :

   - déterminer une première dose de gelsoline G1 dans un échantillon biologique dudit patient issu d'un premier prélèvement au temps T1, effectué entre le jour d'admission en service de réanimation et 48 heures après,
   - déterminer une deuxième dose de gelsoline G2 dans un échantillon biologique dudit patient issu d'un second prélèvement au temps T2, effectué deux à trois jours après le premier prélèvement,
   - calculer la variation entre la dose de gelsoline G2 et la dose de gelsoline G1, donnant une valeur $\Delta$,
   - comparer la valeur $\Delta$ obtenue à la précédente étape, à une valeur seuil S préalablement déterminée à partir de deux populations de patients admis en service de réanimation, l'une n'ayant pas développé une infection disséminée et l'autre ayant développé une telle infection,

     • une valeur $\Delta$ inférieure à ladite valeur seuil S signifiant que le patient admis en service de réanimation est un patient à haut risque de développer une infection disséminée, et
     • une valeur $\Delta$ supérieure à ladite valeur seuil S signifiant que le patient admis en service de réanimation n'est pas un patient à haut risque de développer une infection disséminée.

2. Procédé selon la revendication 1, **caractérisé en ce que** la valeur $\Delta$ est calculée selon la formule suivante (I) :

$$G2 - G1 \text{ (I)}$$

3. Procédé selon la revendication 1, **caractérisé en ce que** la valeur $\Delta$ correspond au taux de variation relative et est calculée selon la formule suivante (II) :

$$\frac{G2 - G1}{G1} \times 100 \text{ (II)}$$

4. Procédé selon la revendication 1, **caractérisé en ce que** la valeur $\Delta$ correspond à la différence de dose par unité de temps et est calculée selon la formule suivante (III) :

$$\frac{G2 - G1}{T2 - T1} \text{ (III)}$$

5. Procédé selon l'une quelconque des revendications 1 à 4, **caractérisé en ce que** le premier prélèvement (T1) est effectué le jour d'admission en service de réanimation.

6. Procédé selon l'une quelconque des revendications 1 à 5, **caractérisé en ce que** les échantillons biologiques sont du sang total, du plasma, du sérum ou tout dérivé de sang.

7. Procédé selon l'une quelconque des revendications 1 à 6, **caractérisé en ce que** la détermination de la dose de gelsoline est réalisée par une technique choisie parmi la spectrométrie de masse et l'immunoessai.

8. Procédé selon l'une quelconque des revendications 1 à 7, **caractérisé en ce que** ledit patient est choisi parmi des patients polytraumatisés, des patients dits grand-brûlés, des patients atteints de pancréatites ou de syndrome respiratoire aigüe.

**Patentansprüche**

1. Verfahren zur Vorhersage des Risikos einer disseminierten Infektion bei einem auf die Intensivstation aufgenommenen Patienten, der kein klinisches Symptom einer solchen Infektion hat, umfassend die folgenden Schritte:

- Bestimmen einer ersten Gelsolin-Dosis G1 in einer biologischen Probe des Patienten, die aus einer ersten Entnahme zum Zeitpunkt T1 stammt, die zwischen dem Tag der Aufnahme auf die Intensivstation und 48 Stunden danach durchgeführt wurde,
- Bestimmen einer zweiten Gelsolin-Dosis G2 in einer biologischen Probe des Patienten, die aus einer zweiten Entnahme zum Zeitpunkt T2 stammt, die zwei bis drei Tage nach der ersten Entnahme durchgeführt wurde,
- Berechnen der Veränderung zwischen der Gelsolin-Dosis G2 und der Gelsolin-Dosis G1, was einen Wert Δ ergibt,
- Vergleichen des im vorhergehenden Schritt erhaltenen Werts Δ mit einem Schwellenwert S, der zuvor ausgehend von zwei auf die Intensivstation aufgenommenen Patientenpopulationen bestimmt wurde, von denen die eine keine disseminierte Infektion entwickelt hat und die andere eine solche Infektion entwickelt hat,

• wobei ein Wert Δ unter dem Schwellenwert S bedeutet, dass der auf die Intensivstation aufgenommene Patient ein Patient mit hohem Risiko, eine disseminierte Infektion zu entwickeln, ist und
• wobei ein Wert Δ über dem Schwellenwert S bedeutet, dass der auf die Intensivstation aufgenommene Patient kein Patient mit hohem Risiko, eine disseminierte Infektion zu entwickeln, ist.

2. Verfahren nach Anspruch 1, **dadurch gekennzeichnet, dass** der Wert Δ nach der folgenden Formel (I) berechnet wird:

$$G2 - G1 \quad (I)$$

3. Verfahren nach Anspruch 1, **dadurch gekennzeichnet, dass** der Wert Δ der Rate der relativen Veränderung entspricht und nach der folgenden Formel (II) berechnet wird:

$$\frac{G2-G1}{G1} \times 100 \quad (II)$$

4. Verfahren nach Anspruch 1, **dadurch gekennzeichnet, dass** der Wert Δ der Dosisdifferenz je Zeiteinheit entspricht und nach der folgenden Formel (III) berechnet wird:

$$\frac{G2-G1}{T2-T1} \quad (III)$$

5. Verfahren nach einem der Ansprüche 1 bis 4, **dadurch gekennzeichnet, dass** die erste Entnahme (T1) am Tag der Aufnahme auf die Intensivstation durchgeführt wird.

6. Verfahren nach einem der Ansprüche 1 bis 5, **dadurch gekennzeichnet, dass** die biologischen Proben Gesamtblut, Plasma, Serum oder jedes Blutderivat sind.

7. Verfahren nach einem der Ansprüche 1 bis 6, **dadurch gekennzeichnet, dass** die Bestimmung der Gelsolin-Dosis mit einem Verfahren ausgeführt wird, das unter der Massenspektrometrie und dem Immunoassay gewählt ist.

8. Verfahren nach einem der Ansprüche 1 bis 7, **dadurch gekennzeichnet, dass** der Patient unter polytramautisierten Patienten, sogenannten schwerstverbrannten Patienten, an Pankreatitis oder an akutem Atemwegssyndrom erkrankten Patienten gewählt ist.

**Claims**

1. A method for predicting the risk of developing a disseminated infection in a patient admitted to intensive care having no clinical symptoms of such an infection, comprising the following steps:

   - determining a first dose of gelsolin G1 in a biological sample from said patient originating from a first sample taken at the time T1, carried out between the day of admission to intensive care and 48 hours afterward,
   - determining a second dose of gelsolin G2 in a biological sample from said patient originating from a second sample taken at the time T2, carried out two to three days after the first sampling,
   - calculating the variation between the dose of gelsolin G2 and the dose of gelsolin G1, giving a Δ value,
   - comparing the Δ value obtained in the preceding step to a threshold value S determined beforehand from two populations of patients admitted to intensive care, one not having developed a disseminated infection and the other having developed such an infection,

     • a Δ value lower than said threshold value S meaning that the patient admitted to intensive care is a patient at high risk of developing a disseminated infection, and
     • a Δ value greater than said threshold value S meaning that the patient admitted to intensive care is not a patient at high risk of developing a disseminated infection.

2. The method as claimed in claim 1, **characterized in that** the Δ value is calculated according to the following formula (I):

$$G2 - G1 \text{ (I)}$$

3. The method as claimed in claim 1, **characterized in that** the Δ value corresponds to the relative rate of change and is calculated according to the following formula (II):

$$\frac{G2 - G1}{G1} \times 100 \text{ (II)}$$

4. The method as claimed in claim 1, **characterized in that** the Δ value corresponds to the difference in dose per unit time and is calculated according to the following formula (III):

$$\frac{G2 - G1}{T2 - T1} \text{ (III)}$$

5. The method as claimed in any one of claims 1 to 4, **characterized in that** the first sampling (T1) is performed on the day of admission to intensive care.

6. The method as claimed in any one of claims 1 to 5, **characterized in that** the biological samples are whole blood, plasma, serum or any blood derivative.

7. The method as claimed in any one of claims 1 to 6, **characterized in that** the dose of gelsolin is determined by a technique chosen from mass spectrometry and immunoassay.

8. The method as claimed in any one of claims 1 to 7, **characterized in that** said patient is selected from multiple-trauma patients, what are referred to as major burns patients, patients suffering from pancreatitis or acute respiratory syndrome.

**FIGURE 1**

FIGURE 2

**FIGURE 3**

**FIGURE 4**

G2 – G1
Test de Mann-Withney p=< .001

FIGURE 5

FIGURE 6

((G2-G1)/G1) * 100
Test de Mann-Withney p=<,001

FIGURE 7

34

FIGURE 8

(G2-G1) / G1 : Courbe ROC

Taux de Vrais Positifs (Sensibilité)

Taux de Faux Positifs (1-Spécificité)

Gelsolin

No discrimination

FIGURE 9

FIGURE 10

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 2781802
- WO 9508000 A
- FR 1554227

**Littérature non-brevet citée dans la description**

- **VINCENT JL.** *Lancet,* 2003
- **EGGIMAN P.** *Chest,* 2001
- **ALI YM et al.** Low-dose recombinant properdin provides substantial protection against Streptococcus pneumonia and Neisseria meningitidis infection. *Proc Natl Acad Sci U.S.A,* 2014, vol. 111 (14), 5301-6
- **ASEHNOUNE K et al.** Hydrocortisone and fludrocortisone for prevention of hospital-acquired pneumonia in patients with severe traumatic brain injury (Corti-TC): a double-blind, multicenter phase 3, randomized placebo-controlled trial. *Lancet Respir Med,* 2014, vol. 2, 706-16
- **BONE R.C. et al.** ACCP/SCCM Consensus Conference Committee. American College of Chest Physicians/Society of Critical Care Medicine. *Chest,* 1992, vol. 101 (6), 1644-1655
- **BRENDAN MACLEAN et al.** *BIOINFORMATICS,* 2010, vol. 26 (7), 966-968
- **BUCKI R. et al.** Inactivation of Endotoxin by Human Plasma Gelsolin. *Biochemistry,* 2005, vol. 44 (28
- **CHAHIN A et al.** The novel immunotherapeutic oligodeoxynucleotide IMT504 protects neutropenic animals from fatal Pseudomonas aeruginosa bacteriemia and sepsis. *Antimicrob Agents Chemother,* 2015, vol. 59 (2), 1225-9
- **EGGIMAN P et al.** Infection control in the ICU. *Chest,* 2001, vol. 120 (6), 2059-93
- **ESSADER AS et al.** A comparison of immobilized pH gradient isoelectric focusing and strong-cation-exchange chromatography as a first dimension in shotgun. *Proteomics,* 2005, vol. 5, 24-34
- **FORTIN T et al.** Clinical Quantitation of Prostate-specific Antigen Biomarker in the Low Nanogram/Milliliter Range by Conventional Bore Liquid Chromatography-Tandem Mass Spectrometry (Multiple Reaction Monitoring) Coupling and Correlation with ELISA Tests. *Mol. Cell Proteomics,* 2009, vol. 8 (5), 1006-1015

- **HANLEY, J.A. ; MCNEIL, B.J.** The meaning and use of the area under a receiver operating characteristic (ROC) curve. *Radiology,* 1982, vol. 143, 29-36
- **JENSEN JU et al.** Procalcitonin-guided interventions against infections to increase early appropriate antibiotics and improve survival in the intensive care unit: a randomized trial. *Crit Care Med,* 2011, vol. 39 (9), 2048-58
- **LAMBERT ML SC et al.** Clinical outcomes of health-care-associated infections and antimicrobial resistance in patients admitted to european intensive-care units : a cohort study. *Lancet Infect Dis,* 2011, vol. 11, 30-8
- **LEE PS et al.** Plasma gelsolin depletion and circulating actin in sepsis: a pilot study. *PLoS One,* 2008, vol. 3 (11), e3712
- **LI GH et al.** Multifunctional roles of gelsolin in health and diseases. *Med Res Rev.,* Septembre 2012, vol. 32 (5), 999-1025
- **MICHEL PE et al.** Protein fractionation in a multicompartment device using Off-Gel, isoelectric focusing. *Electrophoresis,* 2003, vol. 24, 3-11
- **OSCHSNER U.A. et al.** Systematic selection of modified aptamer pairs for diagnostic sandwich assays. *BioTechniques,* 2014, vol. 56, 125-133
- **PUISIEUX F et al.** Prophylactic antibiotherapy using cefapirin in the surgery of duodenal ulcer : a randomized clinical trial. *Ann Fr Anesth Reanim,* 1993, vol. 12 (3), 289-92
- **VINCENT JL.** Nosocomial infections in adult intensive-care units. *Lancet,* 2003, vol. 361 (9374), 2068-77
- **WANG H et al.** Time course of plasma gelsolin concentrations during severe sepsis in critically ill surgical patients. *Crit Care,* 2008, vol. 12 (4), R106
- **XIANHUI L et al.** The association between plasma gelsolin level and prognosis of burn patients. *Burns,* 2014, vol. 40 (8), 1552-1555
- **ZWEIG, M. H. ; CAMPBELL G.** Receiver-Operating Characteristic (ROC) Plots : a fundamental evaluation tool in clinical medicine. *Clin. Chem.,* 1993, vol. 39 (4), 561-577

SEQUENCE LISTING

<110> bioMérieux
HOSPICES CIVILS DE LYON
CENTRE HOSPITALIER UNIVERSITAIRE DE NANTES

<120> Prédiction du risque de développer, pour des patients admis en service de réanimation, une infection disséminée

<130> GELSOLINE

<150> FR1554227
<151> 2015-05-12

<160> 4

<170> PatentIn version 3.5

<210> 1
<211> 782
<212> PRT
<213> Homo sapiens

<400> 1

```
Met Ala Pro His Arg Pro Ala Pro Ala Leu Leu Cys Ala Leu Ser Leu
1               5               10              15

Ala Leu Cys Ala Leu Ser Leu Pro Val Arg Ala Ala Thr Ala Ser Arg
        20              25              30

Gly Ala Ser Gln Ala Gly Ala Pro Gln Gly Arg Val Pro Glu Ala Arg
        35              40              45

Pro Asn Ser Met Val Val Glu His Pro Glu Phe Leu Lys Ala Gly Lys
    50              55              60

Glu Pro Gly Leu Gln Ile Trp Arg Val Glu Lys Phe Asp Leu Val Pro
65              70              75              80

Val Pro Thr Asn Leu Tyr Gly Asp Phe Phe Thr Gly Asp Ala Tyr Val
            85              90              95

Ile Leu Lys Thr Val Gln Leu Arg Asn Gly Asn Leu Gln Tyr Asp Leu
            100             105             110

His Tyr Trp Leu Gly Asn Glu Cys Ser Gln Asp Glu Ser Gly Ala Ala
        115             120             125

Ala Ile Phe Thr Val Gln Leu Asp Asp Tyr Leu Asn Gly Arg Ala Val
    130             135             140

Gln His Arg Glu Val Gln Gly Phe Glu Ser Ala Thr Phe Leu Gly Tyr
145             150             155             160
```

Phe Lys Ser Gly Leu Lys Tyr Lys Lys Gly Gly Val Ala Ser Gly Phe
165                 170                 175

Lys His Val Val Pro Asn Glu Val Val Val Gln Arg Leu Phe Gln Val
180                 185                 190

Lys Gly Arg Arg Val Val Arg Ala Thr Glu Val Pro Val Ser Trp Glu
195                 200                 205

Ser Phe Asn Asn Gly Asp Cys Phe Ile Leu Asp Leu Gly Asn Asn Ile
210                 215                 220

His Gln Trp Cys Gly Ser Asn Ser Asn Arg Tyr Glu Arg Leu Lys Ala
225                 230                 235                 240

Thr Gln Val Ser Lys Gly Ile Arg Asp Asn Glu Arg Ser Gly Arg Ala
245                 250                 255

Arg Val His Val Ser Glu Glu Gly Thr Glu Pro Glu Ala Met Leu Gln
260                 265                 270

Val Leu Gly Pro Lys Pro Ala Leu Pro Ala Gly Thr Glu Asp Thr Ala
275                 280                 285

Lys Glu Asp Ala Ala Asn Arg Lys Leu Ala Lys Leu Tyr Lys Val Ser
290                 295                 300

Asn Gly Ala Gly Thr Met Ser Val Ser Leu Val Ala Asp Glu Asn Pro
305                 310                 315                 320

Phe Ala Gln Gly Ala Leu Lys Ser Glu Asp Cys Phe Ile Leu Asp His
325                 330                 335

Gly Lys Asp Gly Lys Ile Phe Val Trp Lys Gly Lys Gln Ala Asn Thr
340                 345                 350

Glu Glu Arg Lys Ala Ala Leu Lys Thr Ala Ser Asp Phe Ile Thr Lys
355                 360                 365

Met Asp Tyr Pro Lys Gln Thr Gln Val Ser Val Leu Pro Glu Gly Gly
370                 375                 380

Glu Thr Pro Leu Phe Lys Gln Phe Phe Lys Asn Trp Arg Asp Pro Asp
385                 390                 395                 400

Gln Thr Asp Gly Leu Gly Leu Ser Tyr Leu Ser Ser His Ile Ala Asn

22

```
                        405                     410                        415


        Val Glu Arg Val Pro Phe Asp Ala Ala Thr Leu His Thr Ser Thr Ala
                    420             425             430


        Met Ala Ala Gln His Gly Met Asp Asp Asp Gly Thr Gly Gln Lys Gln
                    435             440             445


        Ile Trp Arg Ile Glu Gly Ser Asn Lys Val Pro Val Asp Pro Ala Thr
            450             455             460


        Tyr Gly Gln Phe Tyr Gly Gly Asp Ser Tyr Ile Ile Leu Tyr Asn Tyr
        465             470             475             480


        Arg His Gly Gly Arg Gln Gly Gln Ile Ile Tyr Asn Trp Gln Gly Ala
                    485             490             495


        Gln Ser Thr Gln Asp Glu Val Ala Ala Ser Ala Ile Leu Thr Ala Gln
                    500             505             510


        Leu Asp Glu Glu Leu Gly Gly Thr Pro Val Gln Ser Arg Val Val Gln
                    515             520             525


        Gly Lys Glu Pro Ala His Leu Met Ser Leu Phe Gly Gly Lys Pro Met
                530             535             540


        Ile Ile Tyr Lys Gly Gly Thr Ser Arg Glu Gly Gly Gln Thr Ala Pro
        545             550             555             560


        Ala Ser Thr Arg Leu Phe Gln Val Arg Ala Asn Ser Ala Gly Ala Thr
                    565             570             575


        Arg Ala Val Glu Val Leu Pro Lys Ala Gly Ala Leu Asn Ser Asn Asp
                    580             585             590


        Ala Phe Val Leu Lys Thr Pro Ser Ala Ala Tyr Leu Trp Val Gly Thr
                    595             600             605


        Gly Ala Ser Glu Ala Glu Lys Thr Gly Ala Gln Glu Leu Leu Arg Val
                610             615             620


        Leu Arg Ala Gln Pro Val Gln Val Ala Glu Gly Ser Glu Pro Asp Gly
        625             630             635             640


        Phe Trp Glu Ala Leu Gly Gly Lys Ala Ala Tyr Arg Thr Ser Pro Arg
                    645             650             655
```

23

```
Leu Lys Asp Lys Lys Met Asp Ala His Pro Pro Arg Leu Phe Ala Cys
        660             665         670

Ser Asn Lys Ile Gly Arg Phe Val Ile Glu Glu Val Pro Gly Glu Leu
        675             680         685

Met Gln Glu Asp Leu Ala Thr Asp Asp Val Met Leu Leu Asp Thr Trp
        690             695         700

Asp Gln Val Phe Val Trp Val Gly Lys Asp Ser Gln Glu Glu Glu Lys
705             710             715             720

Thr Glu Ala Leu Thr Ser Ala Lys Arg Tyr Ile Glu Thr Asp Pro Ala
            725             730             735

Asn Arg Asp Arg Arg Thr Pro Ile Thr Val Val Lys Gln Gly Phe Glu
        740             745             750

Pro Pro Ser Phe Val Gly Trp Phe Leu Gly Trp Asp Asp Asp Tyr Trp
        755             760             765

Ser Val Asp Pro Leu Asp Arg Ala Met Ala Glu Leu Ala Ala
770             775             780
```

<210> 2
<211> 11
<212> PRT
<213> Artificial Sequence

<220>
<223> Peptide

<400> 2

```
His Val Val Pro Asn Glu Val Val Val Gln Arg
1               5               10
```

<210> 3
<211> 13
<212> PRT
<213> Artificial Sequence

<220>
<223> Peptide

<400> 3

```
Ala Gly Ala Leu Asn Ser Asn Asp Ala Phe Val Leu Lys
1               5               10
```

<210> 4
<211> 17

24

<212> PRT
<213> Artificial Sequence

<220>
<223> Peptide

<400> 4

```
Gln Thr Gln Val Ser Val Leu Pro Glu Gly Gly Glu Thr Pro Leu Phe
1               5                   10                  15

Lys
```